(19) Europäisches Patentamt
European Patent Office
Office européen des brevets

(11) EP 4 534 558 A1

(12) EUROPEAN PATENT APPLICATION
published in accordance with Art. 153(4) EPC

(43) Date of publication:
**09.04.2025 Bulletin 2025/15**

(21) Application number: **23815061.9**

(22) Date of filing: **25.05.2023**

(51) International Patent Classification (IPC):
***C07K 16/28*** *(2006.01)* ***C07K 14/54*** *(2006.01)*
***C07K 16/18*** *(2006.01)* ***C07K 16/30*** *(2006.01)*
***C12N 15/13*** *(2006.01)* ***A61K 39/395*** *(2006.01)*
***A61K 47/68*** *(2017.01)* ***G01N 33/68*** *(2006.01)*
***G01N 33/574*** *(2006.01)* ***G01N 33/577*** *(2006.01)*
***A61P 35/00*** *(2006.01)*

(52) Cooperative Patent Classification (CPC):
**A61K 39/395; A61K 47/68; A61P 35/00; C07K 14/54; C07K 16/00; C07K 16/18; C07K 16/28; C07K 16/30; G01N 33/574; G01N 33/577; G01N 33/68**

(86) International application number:
**PCT/CN2023/096155**

(87) International publication number:
**WO 2023/231877 (07.12.2023 Gazette 2023/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.05.2022 CN 202210605505**
**22.12.2022 CN 202211657261**
**19.04.2023 CN 202310420427**

(71) Applicant: **Shandong Boan Biotechnology Co., Ltd.**
**Yantai, Shandong 264670 (CN)**

(72) Inventors:
- **SONG, Deyong**
  **Yantai, Shandong 264670 (CN)**
- **HAN, Jing**
  **Yantai, Shandong 264670 (CN)**
- **LIU, Hong**
  **Yantai, Shandong 264670 (CN)**
- **SHEN, Nan**
  **Yantai, Shandong 264670 (CN)**
- **JIAO, Jie**
  **Yantai, Shandong 264670 (CN)**
- **WANG, Qiaoping**
  **Yantai, Shandong 264670 (CN)**
- **FENG, Jianxia**
  **Yantai, Shandong 264670 (CN)**
- **LI, Min**
  **Yantai, Shandong 264670 (CN)**
- **ZONG, Mengqi**
  **Yantai, Shandong 264670 (CN)**
- **LI, Ying**
  **Yantai, Shandong 264670 (CN)**
- **DOU, Changlin**
  **Yantai, Shandong 264670 (CN)**

(74) Representative: **karo IP Patentanwälte PartG mbB**
**Steinstraße 16-18**
**40212 Düsseldorf (DE)**

Remarks:
The complete document including Reference Table(s) and the Sequence Listing(s) can be downloaded from the EPO website

# (54) ANTI-CD228 ANTIBODY AND DRUG CONJUGATE THEREOF

(57) An antibody or an antigen-binding fragment thereof that binds to CD228, and an antibody-drug conjugate containing the antibody or antigen-binding fragment thereof. The antibody or antigen-binding fragment thereof has a high affinity to a CD228 protein and an ability to mediate ADCC. The antibody-drug conjugate exhibits an inhibitory effect on various tumors. A nucleic acid encoding the antibody or antigen-binding fragment thereof; a cell containing the nucleic acid; a pharmaceutical composition containing the antibody or antigen-



Processed by Luminess, 75001 PARIS (FR)

(Cont. next page)

binding fragment thereof, the nucleic acid, the cell or the antibody-drug conjugate; and a kit. The use of the antibody or antigen-binding fragment thereof, the nucleic acid, the antibody-drug conjugate or the pharmaceutical composition in the prevention, treatment, detection or diagnosis of diseases related to CD228.

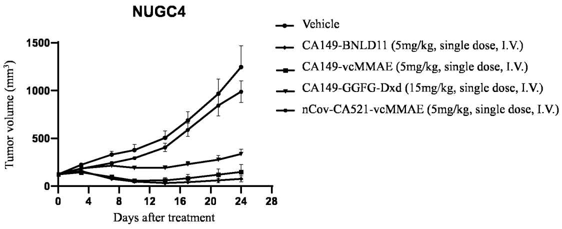

## Description

### TECHNICAL FIELD

The present invention relates to the field of biomedicine or biopharmaceutical technology, and in particular, to an anti-CD228 antibody and a drug conjugate thereof.

### BACKGROUND

CD228 (also known as melanotransferrin, MTF, melanoma-associated antigen p97, MFI2, or MAP97) is a 90-97 kDa asialoglycoprotein member of the transferrin family. CD228 is typically found anchored to the cell membrane via a glycosylphosphatidylinositol anchor, with only a small amount of soluble protein being detectable.

CD228 plays a role in cell proliferation, migration, and tumorigenesis. Increased expression of CD228 may lead to accelerated melanoma tumor growth. In cell models, high expression of CD228 may increase cell proliferation, while downregulation of CD228 expression leads to reduced cell proliferation.

CD228 is expressed in various tumors, including melanoma, mesothelioma, pancreatic cancer, non-small cell lung cancer, breast cancer, and colon cancer, making it broadly applicable. CD228 is expressed in 72% of melanomas and 79% of pancreatic cancers, as well as in 83% of mesotheliomas, 100% of colon cancers, 57% of breast cancers, and 69% of squamous cell carcinomas, exhibiting great clinical demands.

Antibody-drug conjugates are molecules combining an antibody with a micromolecular chemotherapeutic drug via a linker, retaining the high targeting property of the antibody and possessing the full cytotoxicity of the chemotherapy drug to effectively kill tumor cells. Several antibody-drug conjugates have been approved, while many others are in development, indicating that the technology has matured. CD228 is highly expressed in various tumor tissues and has low or no expression in normal tissues. Thus, CD228 may be an ideal antibody-drug conjugate target due to its differential expression.

Therefore, providing a novel CD228 antibody-drug conjugate as an effective anti-cancer drug holds broad application value in the pharmaceutical field.

### SUMMARY

The present invention provides an anti-CD228 antibody or an antigen-binding fragment thereof capable of binding to CD228 protein. The present invention further provides a nucleic acid encoding the antibody or the antigen-binding fragment thereof; a cell comprising the nucleic acid; a pharmaceutical composition comprising the antibody or the antigen-binding fragment thereof, the nucleic acid, or the cell; a kit comprising the antibody or the antigen-binding fragment thereof, the nucleic acid, or the pharmaceutical composition; use of the antibody or the antigen-binding fragment thereof, the nucleic acid, or the pharmaceutical composition in preventing, treating, detecting, or diagnosing a CD228-associated disease, use of the CD228 antibody or the antigen-binding fragment thereof in preparing an antibody-drug conjugate (ADC); and an anti-CD228 antibody conjugate.

In one aspect, the present invention provides an anti-CD228 antibody or an antigen-binding fragment thereof, comprising the following 3 light chain complementarity determining regions and/or 3 heavy chain complementarity determining regions:

the 3 light chain complementarity determining regions of the antibody or the antigen-binding fragment thereof comprise an LCDR1 set forth in SEQ ID NO: 13, an LCDR2 set forth in SEQ ID NO: 14, and an LCDR3 set forth in SEQ ID NO: 15, and/or the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 18;

the 3 light chain complementarity determining regions of the antibody or the antigen-binding fragment thereof comprise an LCDR1 set forth in SEQ ID NO: 19, an LCDR2 set forth in SEQ ID NO: 20, and an LCDR3 set forth in SEQ ID NO: 21, and/or the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 22;

the 3 light chain complementarity determining regions of the antibody or the antigen-binding fragment thereof comprise an LCDR1 set forth in SEQ ID NO: 23, an LCDR2 set forth in SEQ ID NO: 20, and an LCDR3 set forth in SEQ ID NO: 21, and/or the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 22;

the 3 light chain complementarity determining regions of the antibody or the antigen-binding fragment thereof

comprise an LCDR1 set forth in SEQ ID NO: 24, an LCDR2 set forth in SEQ ID NO: 25, and an LCDR3 set forth in SEQ ID NO: 26, and/or the 3 heavy chain complementarity determining regions of the antibody or the antigen-binding fragment thereof comprise an HCDR1 set forth in SEQ ID NO: 16, an HCDR2 set forth in SEQ ID NO: 27, and an HCDR3 set forth in SEQ ID NO: 28;

the 3 light chain complementarity determining regions of the antibody or the antigen-binding fragment thereof comprise an LCDR1 set forth in SEQ ID NO: 29, an LCDR2 set forth in SEQ ID NO: 25, and an LCDR3 set forth in SEQ ID NO: 30, and/or the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 28;

the 3 light chain complementarity determining regions of the antibody or the antigen-binding fragment thereof comprise an LCDR1 set forth in SEQ ID NO: 31, an LCDR2 set forth in SEQ ID NO: 14, and an LCDR3 set forth in SEQ ID NO: 21, and/or the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 32;

the 3 light chain complementarity determining regions of the antibody or the antigen-binding fragment thereof comprise an LCDR1 set forth in SEQ ID NO: 19, an LCDR2 set forth in SEQ ID NO: 44, and an LCDR3 set forth in SEQ ID NO: 45, and/or the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 49; or, the 3 light chain complementarity determining regions of the antibody or the antigen-binding fragment thereof comprise an LCDR1 set forth in SEQ ID NO: 46, an LCDR2 set forth in SEQ ID NO: 47, and an LCDR3 set forth in SEQ ID NO: 48, and/or the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 28.

In one specific embodiment, the present invention provides an anti-CD228 antibody or an antigen-binding fragment thereof, wherein the antibody or the antigen-binding fragment thereof, when binding to CD228, binds to at least one of the following residues set forth in SEQ ID NO: 41: E312A, L313A, R282A, and R275A.

In one specific embodiment, the present invention provides an anti-CD228 antibody or an antigen-binding fragment thereof, wherein:

the antibody or the antigen-binding fragment thereof comprises a light chain variable region set forth in SEQ ID NO: 1 and/or a heavy chain variable region set forth in SEQ ID NO: 2;

the antibody or the antigen-binding fragment thereof comprises a light chain variable region set forth in SEQ ID NO: 3 and/or a heavy chain variable region set forth in SEQ ID NO: 4;

the antibody or the antigen-binding fragment thereof comprises a light chain variable region set forth in SEQ ID NO: 5 and/or a heavy chain variable region set forth in SEQ ID NO: 6;

the antibody or the antigen-binding fragment thereof comprises a light chain variable region set forth in SEQ ID NO: 7 and/or a heavy chain variable region set forth in SEQ ID NO: 8;

the antibody or the antigen-binding fragment thereof comprises a light chain variable region set forth in SEQ ID NO: 9 and/or a heavy chain variable region set forth in SEQ ID NO: 10; the antibody or the antigen-binding fragment thereof comprises a light chain variable region set forth in SEQ ID NO: 11 and/or a heavy chain variable region set forth in SEQ ID NO: 12; the antibody or the antigen-binding fragment thereof comprises a light chain variable region set forth in SEQ ID NO: 38 and/or a heavy chain variable region set forth in SEQ ID NO: 37; or

the antibody or the antigen-binding fragment thereof comprises a light chain variable region set forth in SEQ ID NO: 40 and/or a heavy chain variable region set forth in SEQ ID NO: 39.

In one specific embodiment of the present invention, the heavy chain constant region sequence of the antibody or the antigen-binding fragment thereof is set forth in SEQ ID NO: 33.

Furthermore, the light chain constant region sequence of the antibody or the antigen-binding fragment thereof is set forth in SEQ ID NO: 34.

In embodiments of the present invention, the antibody or the antigen-binding fragment thereof disclosed herein includes a monoclonal antibody, a polyclonal antibody, a chimeric antibody, a humanized antibody, Fab, Fab', $F(ab')_2$, Fv, scFv or dsFv fragment.

In a second aspect, the present invention provides a nucleic acid encoding the antibody or the antigen-binding fragment thereof.

In a third aspect, the present invention provides a vector comprising the nucleic acid encoding the antibody or the antigen-binding fragment thereof. The vector can be used to express the antibody or the antigen-binding fragment thereof. Preferably, the vector may be a viral vector; preferably, the viral vector includes, but is not limited to, a lentivirus vector, an

adenovirus vector, an adeno-associated virus vector, or a retrovirus vector; preferably, the vector may be a non-viral vector; preferably, the vector may be a mammalian expression vector; preferably, the expression vector may be a bacterial expression vector; preferably, the expression vector may be a fungal expression vector.

In a fourth aspect, the present invention provides a cell, wherein the comprises the nucleic acid or the vector, and is capable of expressing the antibody or the antigen-binding fragment thereof. Preferably, the cell is a bacterial cell; preferably, the bacterial cell is an *Escherichia coli* cell or the like; preferably, the cell is a fungal cell; preferably, the fungal cell is a yeast cell; preferably, the yeast cell is a *Pichia pastoris* cell or the like; preferably, the cell is a mammalian cell; and preferably, the mammalian cell is a Chinese hamster ovary (CHO) cell, a human embryonic kidney cell (293), a B cell, a T cell, a DC cell, an NK cell, or the like.

In a fifth aspect, the present invention provides an anti-CD228 antibody conjugate, wherein the anti-CD228 antibody conjugate comprises: (a) the CD228 antibody or the antigen-binding fragment thereof, and (b) a conjugate moiety coupled to the antibody moiety, wherein the conjugate moiety is selected from one or more of a detectable label, a drug, a toxin, a cytokine, a radionuclide, and an enzyme.

In another preferred embodiment, the antibody-drug conjugate (ADC) is represented by formula 1 below:

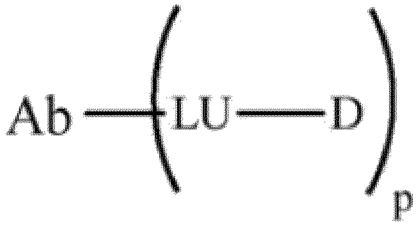

-------Formula 1

wherein, in formula 1: Ab is the anti-CD228 antibody or the antigen-binding fragment thereof disclosed herein, LU is a linker, and D is a drug; the subscript p corresponds to the average DAR value for the antibody-drug conjugate, and is a value selected from 1-10, preferably 1-8, more preferably 1-4 or 4-8, and even more preferably 4.

The drug is selected from a chemotherapeutic agent, a radiotherapeutic agent, a hormone therapeutic agent, or an immunotherapeutic agent. Optionally, the drug is selected from the following group: a taxane, a maytansinoid, a camptothecin, a tubulysin, an auristatin, a calicheamicin, an anthracycline, docetaxel, cathepsin, ricin, gelonin, Pseudomonas exotoxin, diphtheria toxin, a ribonuclease (RNase), or a radioisotope.

Furthermore, linker LU has a general formula R'-L1-L2-L3;
wherein, in the general formula, L3 is:

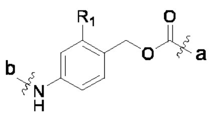 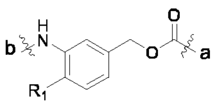 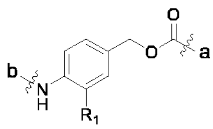 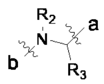 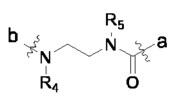 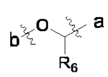

wherein end a of L3 is linked to drug D while end b is linked to L2; $R_1$ is hydrogen, carboxyl, ester group, nitro, sulfonyl, or halogen; or $R_1$ is

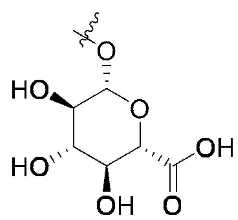

;

$R_2$-$R_6$ are each independently hydrogen,

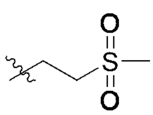 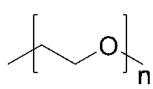

,

n being 0-8; in the general formula, L2 is:

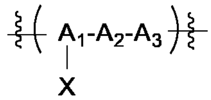, 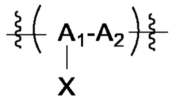, or 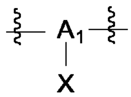,

wherein A is each independently phenylalanine residue, glycine residue, alanine residue, glutamic acid residue, aspartic acid residue, cysteine residue, histidine residue, lysine residue, proline residue, valine, citrulline residue, β-glycine residue, or β-alanine residue; X is:

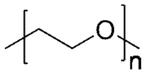 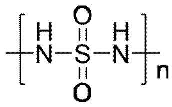 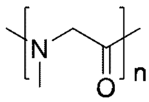,

n being 0-8; in the general formula, L1 is:

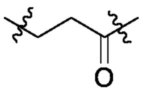 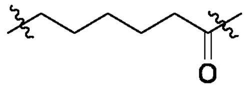 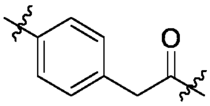 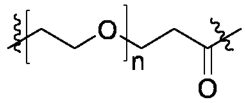;

and/or in the general formula, R' is:

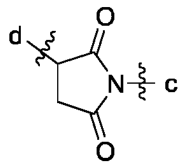 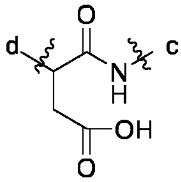,

wherein end c of R' is linked to L1 while end d is linked to A;

in one preferred embodiment, in formula 1 for the antibody-drug conjugate (ADC), LU-D is VcMMAE, wherein LU is Vc (valine-citrulline linker), and D is MMAE (monomethyl auristatin E); VcMMAE may also be written as MC-Val-Cit-PAB-MMAE or mc-vc-PAB-MMAE.

In one preferred embodiment, in formula 1 for the antibody-drug conjugate (ADC), LU-D is BNLD11, wherein LU is MC-β-Ala-(glucuronide) PAB, and D is MMAE; BNLD11 structure is as follows:

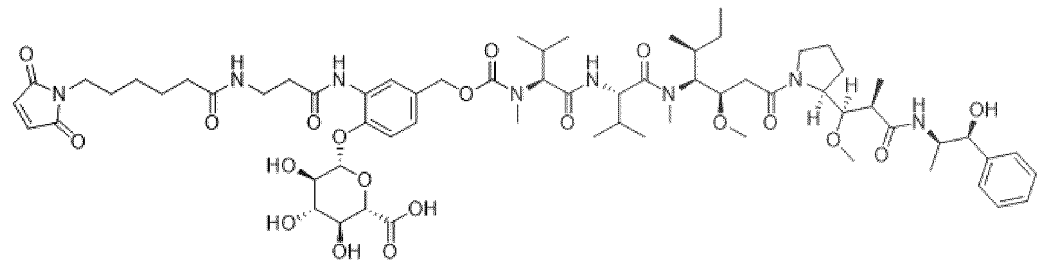,

wherein the exact mass of BNLD11 is 1322.690; BNLD11 is synthesized by a conventional method in the prior art; in one preferred example, BNLD11 is obtained through the synthetic route shown in FIG 30.

In one preferred example, in formula 1 for the ADC, the 3 light chain complementarity determining regions of the anti-CD228 antibody or the antigen-binding fragment thereof (Ab) comprise an LCDR1 set forth in SEQ ID NO: 24, an LCDR2 set forth in SEQ ID NO: 25, and an LCDR3 set forth in SEQ ID NO: 26, and the 3 heavy chain complementarity determining regions of the antibody or the antigen-binding fragment thereof comprise an HCDR1 set forth in SEQ ID NO: 16, an HCDR2 set forth in SEQ ID NO: 27, and an HCDR3 set forth in SEQ ID NO: 28; preferably, the antibody or the antigen-binding fragment thereof comprises a light chain variable region set forth in SEQ ID NO: 7 and a heavy chain variable region set forth in SEQ ID NO: 8; more preferably, the heavy chain constant region sequence of the antibody or the

antigen-binding fragment thereof is set forth in SEQ ID NO 33, and/or the light chain constant region sequence of the antibody or the antigen-binding fragment thereof is set forth in SEQ ID NO 34.

In one preferred example, in formula 1 for the ADC, LU-D is VcMMAE, p is 4, and Ab is an anti-CD228 antibody or an antigen-binding fragment thereof; the 3 light chain complementarity determining regions of the anti-CD228 antibody or the antigen-binding fragment thereof comprise an LCDR1 set forth in SEQ ID NO: 24, an LCDR2 set forth in SEQ ID NO: 25, and an LCDR3 set forth in SEQ ID NO: 26, and the 3 heavy chain complementarity determining regions of the antibody or the antigen-binding fragment thereof comprise an HCDR1 set forth in SEQ ID NO: 16, an HCDR2 set forth in SEQ ID NO: 27, and an HCDR3 set forth in SEQ ID NO: 28; more preferably, the antibody or the antigen-binding fragment thereof comprises a light chain variable region set forth in SEQ ID NO: 7 and a heavy chain variable region set forth in SEQ ID NO: 8; more preferably, the heavy chain constant region sequence of the antibody or the antigen-binding fragment thereof is set forth in SEQ ID NO 33, and/or the light chain constant region sequence of the antibody or the antigen-binding fragment thereof is set forth in SEQ ID NO 34.

In one preferred example, in formula 1 for the ADC, LU-D is BNLD11 structure, and p is 4, wherein BNLD11 structure is as follows:

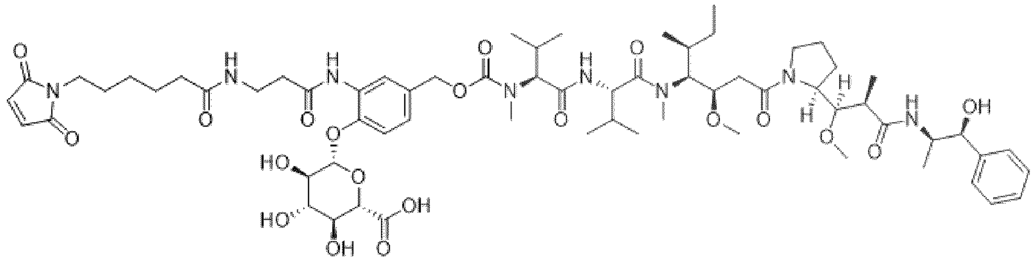

,

and Ab is an anti-CD228 antibody or an antigen-binding fragment thereof; the 3 light chain complementarity determining regions of the anti-CD228 antibody or the antigen-binding fragment thereof comprise an LCDR1 set forth in SEQ ID NO: 24, an LCDR2 set forth in SEQ ID NO: 25, and an LCDR3 set forth in SEQ ID NO: 26, and the 3 heavy chain complementarity determining regions of the antibody or the antigen-binding fragment thereof comprise an HCDR1 set forth in SEQ ID NO: 16, an HCDR2 set forth in SEQ ID NO: 27, and an HCDR3 set forth in SEQ ID NO: 28; more preferably, the antibody or the antigen-binding fragment thereof comprises a light chain variable region set forth in SEQ ID NO: 7 and a heavy chain variable region set forth in SEQ ID NO: 8.

In a sixth aspect, the present invention provides a pharmaceutical composition comprising the antibody or the antigen-binding fragment thereof, the nucleic acid, the vector, the cell, or the antibody-drug conjugate, and preferably the pharmaceutical composition further comprises a pharmaceutically acceptable carrier, wherein preferably the pharmaceutically acceptable carrier comprises one or more of: a pharmaceutically acceptable solvent, a dispersant, an additive, a plasticizer, or other pharmaceutically acceptable excipients.

In a seventh aspect, the present invention provides a kit comprising the antibody or the antigen-binding fragment thereof according to the present invention, or comprising a nucleic acid encoding the antibody or the antigen-binding fragment thereof, the pharmaceutical composition, or the antibody-drug conjugate.

In an eighth aspect, the present invention provides use of the antibody or the antigen-binding fragment thereof, the nucleic acid, the vector, the cell, or the antibody-drug conjugate in preparing a pharmaceutical composition for treating or preventing a disease.

In a ninth aspect, the present invention provides use of the antibody or the antigen-binding fragment thereof, or the nucleic acid in preparing a diagnostic or detection kit.

In a tenth aspect, the present invention provides a method for treating or preventing a disease, comprising administering to a subject in need the antibody or the antigen-binding fragment, the nucleic acid, the vector, the cell, the pharmaceutical composition, or the antibody-drug conjugate disclosed herein.

In an eleventh aspect, the present invention provides a diagnosis or detection method, comprising administering to a subject in need or a sample the antibody or the antigen-binding fragment, the nucleic acid, the kit, or the pharmaceutical composition disclosed herein.

In a twelfth aspect, the present invention provides use of the antibody or the antigen-binding fragment thereof, the nucleic acid, the vector, the cell, the pharmaceutical composition, or the antibody-drug conjugate for treating and preventing a disease.

In a thirteenth aspect, the present invention provides use of the antibody or the antigen-binding fragment thereof, the nucleic acid, the kit, or the pharmaceutical composition in detection and diagnosis.

In a fourteenth aspect, the present invention provides use of the antibody or the antigen-binding fragment thereof, the nucleic acid, the pharmaceutical composition, or the antibody-drug conjugate in preparing a formulation for preventing,

treating, detecting, or diagnosing a CD228-associated disease.

In embodiments of the present invention, the CD228-associated disease includes one or more of melanoma, lung cancer, non-small cell lung cancer, gastric cancer, colon cancer, colon adenocarcinoma, mesothelioma, pancreatic cancer, and breast cancer.

In a fifteenth aspect, the present invention further provides use of the CD228 antibody or the antigen-binding fragment thereof disclosed herein in preparing an antibody-drug conjugate (ADC).

The anti-CD228 antibody and the antibody-drug conjugate thereof disclosed herein have one or more of the following advantages:

1. The anti-CD228 antibody or the antigen-binding fragment thereof disclosed herein has good affinity for CD228 protein and cells expressing CD228 protein.
2. The anti-CD228 antibody-drug conjugate disclosed herein has good killing ability against human melanoma SK-MEL-5 cells.
3. The anti-CD228 antibody-drug conjugate disclosed herein has good cancer inhibition effects in SK-MEL-5, NCI-H226, CALU-1, and NUGC4 animal models, and shows stable pharmacodynamic data for CD228-associated diseases.
4. The anti-CD228 antibody-drug conjugate disclosed herein has good pharmacokinetics in mice.
5. The anti-CD228 antibody-drug conjugate disclosed herein has mild adverse effects and low toxicity, and thus high safety.
6. The anti-CD228 antibody disclosed herein has better internalization and intracellular killing effects than commercial antibodies, such as the CD228-targeted antibody hL49 from Seagen.

## BRIEF DESCRIPTION OF THE DRAWINGS

FIG. 1 shows the expression level of CD228 on TMA chips in Example 1.

FIG. 2 shows the serum titer of CD228-immunized mice in Example 2.

FIG. 3 shows the binding of anti-CD228 antibodies to human CD228 protein in Example 4.

FIG. 4 shows the binding activity of anti-CD228 antibodies to human melanoma SK-MEL-5 cells expressing CD228 protein in Example 5.

FIG. 5A shows the results of internalization assay for anti-CD228 antibodies based on human melanoma SK-MEL-5 cells in Example 6; FIG 5B shows the results of internalization assay for anti-CD228 antibodies based on human lung cancer A549-CD228 cells in Example 6.

FIG. 6 shows the results of ADCC assay for anti-CD228 antibodies based on human melanoma SK-MEL-5 cells in Example 7.

FIG. 7 shows a schematic illustrating the results of sequence comparison between full-length hCD228 and soluble antigen sMFI2 in Example 8.

FIG. 8 shows a HIC-HPLC chromatogram of CA149-BNLD11 in Example 9.

FIG. 9A shows the results of killing effect assay of CA13-VcMMAE against human melanoma SK-MEL-5 cells in Example 10. FIG. 9B shows the results of killing effect assay of CA67-VcMMAE against human melanoma SK-MEL-5 cells in Example 10. FIG 9C shows the results of killing effect assay of CA149-VcMMAE against human melanoma SK-MEL-5 cells in Example 10. FIG. 9D shows the results of killing effect assay of BA352-VcMMAE against human melanoma SK-MEL-5 cells in Example 10. FIG 9E shows the results of killing effect assay of CA518-VcMMAE against human melanoma SK-MEL-5 cells in Example 10. FIG. 9F shows the results of killing effect assay of CA185-VcMMAE against human melanoma SK-MEL-5 cells in Example 10.

FIG. 10 shows the pharmacodynamic data of anti-CD228 ADCs (3 mg/kg) on an SK-MEL-5 animal model.

FIG. 11 shows the pharmacodynamic data of anti-CD228 ADCs (5 mg/kg) on an SK-MEL-5 animal model.

FIG. 12 shows the pharmacodynamic data of anti-CD228 ADCs (3 mg/kg) on the NCI-H226 animal model in Example 11.

FIG. 13 shows the pharmacodynamic data of anti-CD228 ADCs (5 mg/kg) on the NCI-H226 animal model in Example 11.

FIG. 14 shows the pharmacokinetic profiles of anti-CD228 ADCs in mice in Example 12.

FIG. 15A shows the inhibitory activity assessment of CA149-BNLD11 against MC38-CD228 cell proliferation in Example 13. FIG. 15B shows the inhibitory activity assessment of CA149-BNLD11 against A375-CD228 cell proliferation in Example 13. FIG. 15C shows the inhibitory activity assessment of CA149-BNLD11 against SK-MEL-5 cell proliferation in Example 13. FIG 15D shows the inhibitory activity assessment of CA149-BNLD11 against A549-CD228 cell proliferation in Example 13. FIG 15E shows the inhibitory activity assessment of CA149-BNLD11

against A375-CD228 cell proliferation in Example 13.

FIG. 16 shows the tumor growth inhibition curves of the antibody-drug conjugates in the Calu-1 lung cancer model in Example 14.

FIG. 17 shows the tumor weight column chart in antibody-drug conjugate treatment groups in the Calu-1 lung cancer model in Example 14.

FIG. 18 shows the tumor growth inhibition curves in antibody-drug conjugate treatment groups in the human melanoma cell SK-MEL-5 nude mice xenograft tumor model in Example 15.

FIG. 19 shows the tumor weight column chart in antibody-drug conjugate treatment groups in the human melanoma cell SK-MEL-5 nude mice xenograft tumor model in Example 15.

FIG. 20 shows the tumor growth inhibition curves in antibody-drug conjugate treatment groups in the human gastric cancer cell NUGC4 Balb/c nude mice xenograft tumor model in Example 16.

FIG. 21 shows the tumor weight column chart in antibody-drug conjugate treatment groups in the human gastric cancer cell NUGC4 Balb/c nude mice xenograft tumor model in Example 16.

FIG. 22 shows the tumor volume growth inhibition curves in the human squamous cell lung cancer cell NCI-H226 Balb/c nude mice xenograft tumor in Example 17.

FIG. 23 shows the tumor volume growth inhibition curves in the human melanoma cell SK-MEL-5 Balb/c nude mice xenograft tumor in Example 18.

FIG. 24 shows the tumor weight growth curves in the human melanoma cell SK-MEL-5 Balb/c nude mice xenograft tumor in Example 18.

FIG. 25 shows the metabolic profiles of antibody-drug conjugate CA149-BNLD11 in mice in Example 19.

FIG. 26 shows the toxicity study of antibody-drug conjugate CA149-BNLD 11 in male mice in Example 20.

FIG. 27 shows the toxicity study of antibody-drug conjugate CA149-BNLD11 in female mice in Example 20.

FIG. 28 shows the body weight changes in cynomolgus monkeys in the medium-dose group (6 mg/kg) and in the high-dose group (10 mg/kg) in Example 21.

FIG. 29 shows the toxicokinetic assay after the first dose in the medium-dose group (6 mg/kg) and in the high-dose group (10 mg/kg) in Example 21.

FIG. 30 shows a schematic of BNLD11 synthetic route.

## DETAILED DESCRIPTION

The present invention will be further illustrated in conjunction with the following specific examples. The examples described herein are only some, but not all, of the examples of the present invention. It will be appreciated that the following examples are intended to provide those of ordinary skills in the art a complete disclosure and description of how to utilize the methods and the compositions, rather than limit the scope of the present invention. Based on the examples of the present invention, all other examples obtained by those of ordinary skills in the art without creative work shall fall within the protection scope of the present invention.

Example 1. CD228 expression assay

PDX sample chips for gallbladder cancer, colon cancer, breast cancer, melanoma, lung cancer, bile duct cancer, pancreatic cancer, cervical cancer, sarcoma, esophageal cancer, and gastric cancer were purchased from Crown Bioscience. Unstained tissue sections from 12 mesothelioma patients were purchased from Shanghai LIDE Biotech., Co. Ltd. (4 patients) and Shanghai Xinchao Biotechnology Co., Ltd. (8 patients). Crown Bioscience was entrusted with the CD228 immunohistochemical staining of all the PDX sample tissue chips and the unstained mesothelioma tissue sections of the 8 patients purchased from Shanghai Xinchao Biotechnology Co., Ltd. Shanghai LIDE Biotech., Co. Ltd. was entrusted with the CD228 immunohistochemical staining of the unstained mesothelioma tissue sections of the 4 patients. CD228 immunohistochemical antibodies were purchased from Novus Biologicals (Catalog No. NBP1-85777) with a primary antibody dilution factor of 1:200.

The immunohistochemical assay was conducted by an IHC automated staining system (Bond RX automatic IHC&ISH system, Leica). Immunohistochemical staining results were evaluated by H-score values.

$$\text{H-score}=\sum[(pi\times i)],$$

wherein pi represents the percentage of positive cells, and i represents the staining intensity (0: negative; 1: weak staining; 2: medium staining; 3: strong staining). Each sample was independently scored thrice, and the average of the three scores was taken as the final H-score value. The IHC scoring results of all samples are shown in FIG. 1. The expression level of CD228 on the TMA chip shown in FIG. 1 and the expression rate of CD228 shown in Table 1 indicate a relatively high

proportion of moderate to high expression of CD228 in gallbladder cancer, colon cancer, breast cancer, melanoma, lung cancer, bile duct cancer, pancreatic cancer, and cervical cancer.

Table 1. Summary of CD228 expression levels in various tumors

| Indication | CD228 expression level (H-score) | | | |
|---|---|---|---|---|
| | 300-200 | 100-200 | 0-100 | 0 |
| Gallbladder cancer | 30.77% | 53.85% | 15.38% | 0% |
| Colon cancer | 3.13% | 71.88% | 3.13% | 21.88% |
| Breast cancer | 44.44% | 27.78% | 0% | 27.78% |
| Melanoma | 50% | 18.75% | 25% | 6.25% |
| Lung cancer | 9.38% | 50% | 15.63% | 25% |
| Bile duct cancer | 21.43% | 35.71% | 42.86% | 0% |
| Pancreatic cancer | 0% | 53.12% | 46.88% | 0% |
| Cervical cancer | 20% | 32% | 44% | 4% |
| Sarcoma | 20% | 16.67% | 50% | 13.33% |
| Esophageal cancer | 10% | 20% | 56.67% | 13.33% |
| Mesothelioma | 16.67% | 8.33% | 66.67% | 8.33% |
| Gastric cancer | 0% | 0% | 56.25% | 43.75% |

**Example 2. Production of anti-CD228 monoclonal antibodies**

**1.1 Protein production**

Jiangsu GenScript Biotech Corporation was entrusted with the gene synthesis of the three proteins in Table 2. CHO cells were transfected and incubated on a shaker at 37 °C/8% $CO_2$/125 rpm. 10 days after the transient transfection, the supernatant was collected. The expression supernatant was purified on a Ni column (GenScript Biotech, L00250), and then subjected to polishing purification on an SP column (GE, 17-1087-01) to give human CD228, mouse CD228 and monkey CD228 proteins.

Table 2. Amino acid sequences of three proteins

| Protein ID | Amino acid sequence |
| --- | --- |
| Human CD228 | MRGPSGALWLLLALRTVLGGMEVRWCATSDPEQHKCGNMSEAFREAGIQPSLLCVRGTSADHCVQLIAAQEADAITLDGGAIYEAGKEHGLKPVVGEVYDQEVGTSYYAVAVVRRSSHVTIDTLKGVKSCHTGINRTVGWNVPVGYLVESGRLSVMGCDVLKAVSDYFGGSCVPGAGETSYSESLCRLCRGDSSGEGVCDKSPLERYYDYSGAFRCLAEGAGDVAFVKHSTVLENTDGKTLPSWGQALLSQDFELLCRDGSRADVTEWRQCHLARVPAHAVVVRADTDGGLIFRLLNEGQRLFSHEGSSFQMFSSEAYGQKDLLFKDSTSELVPIATQTYEAWLGHEYLHAMKGLLCDPNRLPPYLRWCVLSTPEIQKCGDMAVAFRRQRLKPEIQCVSAKSPQHCMERIQAEQVDAVTLSGEDIYTAGKTYGLVPAAGEHYAPEDSSNSYYVVAVVRRDSSHAFTLDELRGKRSCHAGFGSPAGWDVPVGALIQRGFIRPKDCDVLTAVSEFFNASCVPVNNPKNYPSSLCALCVGDEQGRNKCVGNSQERYYGYRGAFRCLVENAGDVAFVRHTTVFDNTNGHNSEPWAAELRSEDYELLCPNGARAEVSQFAACNLAQIPPHAVMVRPDTNIFTVYGLLDKAQDLFGDDHNKNGFKMFDSSNYHGQDLLFKDATVRAVPVGEKTTYRGWLGLDYVAALEGMSSQQHHHHHHHHHH(SEQ ID NO:41) |
| Mouse CD228 | MRLLSVTFWLLLSLRTVLCVMEMQWCTISDPEQQKCKDMSKAFQGAGIQPSLLCVQGTSTDHCVQLIKDQKADAITLDGGAIYQAGKEYGLKPVVGEVYDQDIGTSYYAVAVVRRNSNVTINTLKGVKSCHTGINRTVGWNVPVGYLVETGHLSVMGCDVLKAVGDYFGGSCVPGAGETSHSESLCRLCRGDSSGHNVCDKSPLERYYDYSGAFRCLAEGAGDVAFVKHSTVLENTDGKTLPSWGKALMSQDFQLLCRDGSRADITEWRRCHLAKVPAHAVVVRDDMDGGLIFRLLNEGQLLFSHEDSSFQMFSSKAYGQKNLLFKDSTLELVPIATQNYESWLGQEYLHAMKGLLCDPNRLPHYLRWCVLSVPEIQKCGDMAVAFSRQSLKPEIQCVSAESPEHCMQQIQAGHIDAVTLRGEDIYRAGKAYGLVPAAGELYAEEDRSNSYFVVAVVRRDSSYSFTLDELRSKRSCHPGLGSPAGWEVPIGSLIQRGFIRPKDCDVLTAVSEFFNASCVPVNNPKNYPSSLCALCVGDEKGRNKCVGSSQERYYGYSGAFRCLVENAGDVAFLKHTTVFENTNGHNPEPWASHLRWQDYELLCPNGARAEVDQFQACNLAQMPSHAVMVHPDTNIFTVYGLLDKAQDLFGDDHNKNGFQMFDSSKYHSQDLLFKDATVRAVPVREKTTYLDWLGPDYVVALEGMLSQQHHHHHHHHHH(SEQ ID NO:42) |

(continued)

| Protein ID | Amino acid sequence |
|---|---|
| Monkey CD228 | MRGPSRALWLLLALRTVLGGMEVRWCVTSDPEQHKCSNMSTAFREAGIQPSLLCVQGTSP DHCIQLIAAQEADAITLDGGAIYEAGKEHGLKPVVGEVYDQEVGTSYYAVAVVKRSSQVTI NTLKGVKSCHTGINRTVGWNVPVGYLVESGRLSVMGCDVLKAVSDYFGGSCVPGAGETRY SESLCRLCRGDSSGEGVCDKSPLERYYDYSGAFRCLVEGAGDVAFVKHSTVLENTDGKTLP SWGQALLSQDFELLCRDGSRADVTEWRQCHLARVPAHAVVVRADTDGGLIFRLLNEGQRL FSHEGSSFQMFSSEAYGQKDLLFKDSTSELVPIATQTYEAWLGQEYLHAMKGLLCDPNRLPP YLRWCVLSTPEIQKCGDMAVAFGRQQLKPEIQCVSAKSPQHCMEQIQAGQIDTVTLSGEDIY TAGKTYGLAPAAGEGYASEDSSNSYFVVAVVRRDSSHAFTLDELWGKRSCHAGFGSPAGW DIPVGALIRRGFIRPKDCDVLTAVSEFFNASCVPVNNPKNYPSSLCALCVGDEQGRNKCVGN SQERYYGNSGAFRCLVENAGDVAFVRHTTVFDNTNGHNSEPWAAELRSEDYELLCPNGAR AEVSQFAACNLAQMPPHAVMVRPDTNIFTVYGLLDKAQDLFGDDHNKNGFKMFDSSNYH GQDLLFKDATVRAVPVGEKTTYRDWLGLDYVAALEGMLSQQHHHHHHHHHH(SEQ ID NO:43) |

**1.2** Mouse immunization procedures

The mice used for immunization were full human antibody-transgenic mice developed by Shandong Boan Biotech Co., Ltd. (with a total of 10 mice immunized). The immunization was performed using an antigenic protein CD228 (0.23 mg/mL, Boan, 20200924, SEQ ID NO: 41) prepared by Shandong Boan Biotechnology Co., Ltd. The immunization was conducted by multiple subcutaneous injections in the abdominal area and inguinal region, with an immunization dose of 20 μg/mouse. The antigen was emulsified in complete Freund's adjuvant for the first immunization and in incomplete Freund's adjuvant for the second to fourth immunizations. The first batch of mice received 3 immunizations and one booster immunization, while the second batch of mice received 4 immunizations and one booster immunization. The immunization doses were given at an interval of 14 days. Starting from the second immunization, peripheral blood serum was collected on day 7 after each immunization to measure antibody titers and exclude mice with ineligible titers. After the mice were immunized, the results of the serum titer detection were shown in FIG. 2, where 2500X, 12500X, and 62500X denote the dilution factor. 3 days after the booster immunization, the mice were euthanized, and the spleen was collected and prepared into single cells for library construction.

1.3 Construction of phage library

After the mice were euthanized and dissected, the spleen was collected, ground with the rubber piston of a syringe, and filtered through a filter membrane. The filtered spleen cells were frozen. RNA was then extracted to prepare cDNA. The construction of phage libraries was conducted according to conventional methods. The library capacity data of the constructed libraries are shown in Table 3.

Table 3. Library capacity of constructed phage libraries for immunized mice

| Name | Q1 | Q2 | Q3 | Q4 |
|---|---|---|---|---|
| Library capacity (n) | $1.2\times10^9$ | $1.4\times10^9$ | $1.2\times10^9$ | $1.2\times10^9$ |
| Name | Q5 | Q8 | Q9 | Q10 |
| Library capacity (n) | $1.1\times10^9$ | $1.5\times10^9$ | $2.2\times10^9$ | $1.0\times10^9$ |

**1.4 Screening by two methods**

1.4.1 Plate screening: Plates were coated with CD228-His protein (prepared in-house). The next day, the phage libraries were added and incubated for 2 h. After washing 4-10 times, the specifically bound phages were eluted with an eluent buffer.

1.44.2 Magnetic bead screening: The CD228-His protein was biotinylated following the instructions of the kit, and then bound with Thermo magnetic beads. After blocking with BSA, the beads were incubated with the phage libraries for 2 h. After washing 4-10 times, specifically bound phages were eluted with an eluent buffer. The antibody clones obtained from the screening and their sources are shown in Table 4.

Table 4. Source of anti-CD228 antibodies obtained by screening

| Clone | Source library | Source mouse | Screening method |
|---|---|---|---|
| CA13 | C228-Q1 | Q1 | Plate screening |
| CA67 | C228-Q1 | Q1 | Plate screening |
| CA149 | C228-Q3 | Q3 | Plate screening |
| CA185 | C228-Q3 | Q3 | Plate screening |
| BA352 | C228-Q1 | Q1 | Magnetic bead screening |
| CA518 | C228-Q1 | Q1 | Plate screening |
| CA523 | C228-Q3 | Q3 | Plate screening |
| CA579 | C228-Q3 | Q3 | Plate screening |

**Example 3. Construction and production of intact antibodies**

133 positive IgG1 clones were constructed and sequenced. The amino acid sequences of the variable regions of 8 lead antibodies are shown in Table 5 below (with CDRs underlined according to IMGT system). The variable region sequences of the antibodies in the examples of the present application are shown in Table 5, and the heavy and light chain constant region sequences are shown in Table 6.

Table 5. Variable region amino acid sequences of 8 antibodies

| Antibody ID | Light chain variable region sequence | Heavy chain variable region sequence |
|---|---|---|
| BA352 | DIQMTQSPSAMSASVGDRVTITCRASQGINNYLAWFQQKPGKVPKRLIYAASSLQSGVPSRFSGSGSGTEFTLTISSLQPEDFATYYCLQHNSYPFTFGPGTKVDIK (SEQ ID NO:1)<br>CDR<br>QGINNY (SEQ ID NO:13)<br>AASS (SEQ ID NO:14)<br>LQHNSYPFT (SEQ ID NO:15) | EVQLVESGGGVVQPGRSLRLSCAASGFTFSSYAMHWVRQAPGKGLEWVAVISYDGSNKYYADSVKGRFTISRDNSKNTLYLQMNSLRAEDTAVYYCARGPYLAAAGTALTFDIWGQGTMVTVSS (SEQ ID NO:2)<br>CDR<br>GFTFSSYA (SEQ ID NO:16)<br>ISYDGSNK (SEQ ID NO:17)<br>ARGPYLAAAGTALTFDI (SEQ ID NO:18) |
| CA13 | DIVMTQSPSSLSASVGDRVTITCRASQGISNYLAWFQQKPGKVPKLLIYAASTLQSGVPSRFSGSGSGTDFTLTISSLQPEDVATYYCQKYNSAPFTFGPGTKVDIK (SEQ ID NO:3)<br>CDR<br>QGISNY (SEQ ID NO:19)<br>AAST (SEQ ID NO:20)<br>QKYNSAPFTF (SEQ ID NO:21) | EVQLVESGGGVVQPGRSLRLSCAASGFTFSSYAMHWVRQAPGKGLEWVAVISYDGSNKYYADSVKGRFTISRDNSKNTLFLQMNSLRAEDTAVYYCARDVYHYGSRSPYYYGMDVWGQGTTVTVSS (SEQ ID NO:4)<br>CDR<br>GFTFSSYA (SEQ ID NO:16)<br>ISYDGSNK (SEQ ID NO:17)<br>ARDVYHYGSRSPYYYGMDV (SEQ ID NO:22) |
| CA67 | DIQMTQSPSSLSASVGDRVTITCRASQAISNYLAWYQQKPGKVPKLLIYAASTLHPGVPSRFSGSGSGTDFTLTISSLQPEDVATYYCQKYNSAPFTFGPGTKVDIK (SEQ ID NO:5)<br>CDR<br>QAISNY (SEQ ID NO:23)<br>AAST (SEQ ID NO:20)<br>QKYNSAPFTF (SEQ ID NO:21) | EVQLVESGGGVVQPGRSLRLSCAASGFTFSSYAMHWVRQAPGKGLEWVAVISYDGSNKYYADSVKGRFTISRDNSKNTLYLQMNSLRAEDTAVYYCARDVYHYGSRSPYYYGMDVWGQGTTVTVSS (SEQ ID NO:6)<br>CDR<br>GFTFSSYA (SEQ ID NO:16)<br>ISYDGSNK (SEQ ID NO:17)<br>ARDVYHYGSRSPYYYGMDV (SEQ ID NO:22) |
| CA149 | EIVLTQSPATLSVSPGERATLSCRASQSVSSNLAWYQQKPGQAPRHLIDGASSRASGIPDRFSGSGSGTDFTLTISRLEPEDFAVYYCQQYGSSPPFTFGPGTKVDIK (SEQ ID NO:7)<br>CDR<br>QSVSSN (SEQ ID NO:24) | EVQLVESGGGVVQPGRSLRLSCAASGFTFSSYAMHWVRQAPGKGLEWVAVISFDGSNKYYTDSVKGRFTISRDNSKNTLYLQMNSLRAEDTAVYYCAREVPYYYGSGPFDYWGQGTLVTVSS (SEQ ID NO:8)<br>CDR |

(continued)

| Antibody ID | Light chain variable region sequence | Heavy chain variable region sequence |
|---|---|---|
| | GASS (SEQ ID NO:25)<br>QQYGSSPPFTF (SEQ ID NO:26) | GFTFSSYA (SEQ ID NO:16)<br>ISFDGSNK (SEQ ID NO:27)<br>AREVPYYYGSGPFDY (SEQ ID NO:28) |
| CA185 | EIVLTQSPGTLSLSPGERATLSCRASQSVSSSSLAWYQQKPGQAPRHLIDGASSRASGIPDRFSGSGSGTDFTLTISRLEPEDFAVYYCQQYGSSYTFGQGTKVDIK (SEQ ID NO:9)<br>CDR<br>QSVSSSS (SEQ ID NO:29)<br>GASS (SEQ ID NO:25)<br>QQYGSSYTF (SEQ ID NO:30) | EVQLVESGGGVVQPGRSLRLSCAASGFTFSSYAMHWVRQAPGKGLEWVAVISYDGSNKYYADSVKGRFTISRDNSKNTLYLQMNSLRAEDTAVYYCAREVPYYYGSGPFDYWGQGTLVTVSS (SEQ ID NO:10)<br>CDR<br>GFTFSSYA (SEQ ID NO:16)<br>ISYDGSNK (SEQ ID NO:17)<br>AREVPYYYGSGPFDY (SEQ ID NO:28) |
| CA518 | DIQMTQSPSSVSASVGDRVTITCRASQGISSWLAWYQQKPGKAPKLLIYAASSLQSGVPSRFSGSGSGTDFTLTISSLQPEDVATYYCQKYNSAPFTFGPGTKVDIK (SEQ ID NO:11)<br>CDR<br>QGISSW (SEQ ID NO:31)<br>AASS (SEQ ID NO:14)<br>QKYNSAPFTF (SEQ ID NO:21) | EVQLVESGGGVVQPGRSLRLSCAASGFTFSSYAMHWVRQAPGKGLEWVAVISYDGSNKYHADSVKGRFTISRDNSKNTLFLQMNSLRAEDTAVYYCVRDVYHYGSRSPYYYGMDVWGQGTTVTVSS (SEQ ID NO:12)<br>CDR<br>GFTFSSYA (SEQ ID NO:16)<br>ISYDGSNK (SEQ ID NO:17)<br>VRDVYHYGSRSPYYYGMDV (SEQ ID NO:32) |
| CA523 | DIQMTQSPSSLSASVGDRVTITCRASQGISNYLAWYQQKPGKVPELLIYAASTLLSGVPSRFGGSGSGTDFTLTISSLQPEDIATYYCQKYNSAPFTFGPGTKVDIK(SEQ ID NO:38)<br><br>QGISNY(SEQ ID NO:19)<br>AAS(SEQ ID NO:44)<br>QKYNSAPFT(SEQ ID NO:45) | EVQLVESGGGVVQPGRSLRLSCAASGFTFSSYAMHWVRQAPGKGLEWVAVISYDGSNKYYADSVKGRFTISRDNSKNTLFLQMNSLRAEDTAVYYCARDVYHYGSRSPYYYGMDVWGQGTTVTVSS(SEQ ID NO:37)<br><br>GFTFSSYA(SEQ ID NO:16)<br>ISYDGSNK(SEQ ID NO:17)<br>ARDVYHYGSRSPYYYGMDV(SEQ ID NO:49) |
| CA579 | DIQLTQSPSTLSASVGDRVTITCRASQSVSSSYLAWYQQKPGQAPRRLIDGASSRATGIPDRFSGSGSGTDFTLTISRLEPEDFAVYYCQHYGSSYTFGQGTKVEIK(SEQ ID NO:40)<br>QSVSSSY(SEQ ID NO:46)<br>GAS(SEQ ID NO:47)<br>QHYGSSYT(SEQ ID NO:48) | EVQLVESGGGVVQPGRSLRLSCAASGFTFSSYAMHWVRQAPGKGLEWVAVISYDGSNKYYADSVKGRFTISRDNSKNTLYLQMNSLRAEDTAVYYCAREVPYYYGSGPFDYWGQGTLVTVSS(SEQ ID NO:39)<br>GFTFSSYA(SEQ ID NO:16)<br>ISYDGSNK(SEQ ID NO:17)<br>AREVPYYYGSGPFDY(SEQ ID NO:28) |

The antibody variable region gene was amplified by molecular biology technique PCR (2× Phanta Max Master Mix, manufacturer: Vazyme, Cat. No.: P515-P1-AA, lot no.: 7E512E1). The heavy and light chain variable region genes of

the antibody were respectively connected with vector pCDNA3.4 (Life Technology) having the nucleic acid sequence of an antibody heavy chain constant region and vector pCDNA3.4 having the nucleic acid sequence of an antibody light chain constant region through homologous recombination.

Table 6. Antibody heavy and light chain constant region amino acid sequences

| |
|---|
| Heavy chain constant region sequence: |
| ASTKGPSVFPLAPSSKSTSGGTAALGCLVKDYFPEPVTVSWNSGALTSGVHTFPAVLQSSGLYSLSSVVTV<br>PSSS<br>LGTQTYICNVNHKPSNTKVDKKVEPKSCDKTHTCPPCPAPELLGGPSVFLFPPKPKDTLMISRTPEVTCVV |
| VDV<br>SHEDPEVKFNWYVDGVEVHNAKTKPREEQYNSTYRVVSVLTVLHQDWLNGKEYKCKVSNKALPAPIE<br>KTISKA<br>KGQPREPQVYTLPPSRDELTKNQVSLTCLVKGFYPSDIAVEWESNGQPENNYKTTPPVLDSDGSFFLYSKL<br>TVDK<br>SRWQQGNVFSCSVMHEALHNHYTQKSLSLSPGK(SEQ ID NO:33) |
| Light chain constant region sequence: |
| RTVAAPSVFIFPPSDEQLKSGTASVVCLLNNFYPREAKVQWKVDNALQSGNSQESVTEQDSKDSTYSLSS<br>TLT<br>LSKADYEKHKVYACEVTHQGLSSPVTKSFNRGEC(SEQ ID NO:34) |

HEK293 cells were transfected with plasmids isolated from sequenced positive clones and incubated at 37 °C/8% $CO_2$/125 rpm on a shaker. 7 days after the transient transfection, the supernatant was purified by Protein A affinity chromatography to give the antibodies. The antibody concentration was determined by UV280 in conjunction with the theoretical extinction coefficient.

The reference antibody Hl49 was synthesized according to the sequence in Patent No. US20200246479A1, and the amino acid sequences are shown in Table 7 below.

Table 7. Amino acid sequences of reference antibody hL49

| | |
|---|---|
| hL49-H | QVQLQESGPG LVKPSETLSL TCTVSGDSIT SGYWNWIRQP PGKGLEYIGY ISDSGITYYN PSLKSRVTIS RDTSKNQYSL KLSSVTAADT AVYYCARRTL ATYYAMDYWG QGTLVTVSSA STKGPSVFPL APSSKSTSGG TAALGCLVKD YFPEPVTVSW NSGALTSGVH TFPAVLQSSG LYSLSSVVTV PSSSLGTQTY ICNVNHKPSN TKVDKKVEPK SCDKTHTCPP CPAPELLGGP SVFLFPPKPK DTLMISRTPE VTCVVVDVSH EDPEVKFNWY VDGVEVHNAK TKPREEQYNS TYRVVSVLTV LHQDWLNGKE YKCKVSNKAL PAPIEKTISK AKGQPREPQV YTLPPSRDEL TKNQVSLTCL VKGFYPSDIA VEWESNGQPE NNYKTTPPVL DSDGSFFLYS KLTVDKSRWQ QGNVFSCSVM HEALHNHYTQ KSLSLSPG(SEQ ID NO:35) |
| hL49-L | DFVMTQSPLS LPVTLGQPAS ISCRASQSLV HSDGNTYLHW YQQRPGQSPR LLIYRVSNRF SGVPDRFSGS GSGTDFTLKI SRVEAEDVGV YYCSQSTHVP PTFGQGTKLE IKRTVAAPSV FIFPPSDEQL KSGTASVVCL LNNFYPREAK VQWKVDNALQ SGNSQESVTE QDSKDSTYSL SSTLTLSKAD YEKHKVYACE VTHQGLSSPV TKSFNRGEC(SEQ ID NO:36) |

**Example 4. Characterization of anti-CD228 monoclonal antibodies**

**4.1 Binding of anti-hCD228 antibody to human CD228 protein**

Human CD228 protein (prepared by Boan, 20201014, SEQ ID NO: 41) was diluted to 0.1 μg/mL with a carbonate-buffered saline (hereinafter referred to as CBS) at pH 9.6. Microplates were coated with the protein at 100 μL/well, and incubated at 4 °C overnight. After washing, the microplates were blocked with skimmed milk powder. The plates were washed, before antibodies diluted with PBST (phosphate-buffered saline, Solarbio P1010, +0.05% Tween20) were added at 100 μL/well. The diluted antibodies were intact antibodies comprising Fc, Fab and the constant region serially 3-fold diluted from 0.1 μg/mL to 8 concentrations with PBST. After washing the plates, goat anti-human IgG (H+L)/HRP (diluted at 1:5000, KPL, 474-1006) was added at 100 μL/well, and the plates were incubated at 37 °C for 1 h. After washing the plates, TMB (Beijing Makewonderbio, 1001) was added at 100 μL/well for chromogenesis, and after 10 min, 50 μL of 2 M $H_2SO_4$ was added to stop the chromogenesis. OD450 was measured on a microplate reader. The antibody hL49 targeting CD228 from Seagen was used as a reference antibody in this study and the following studies. FIG. 3 shows the binding curves of the antibodies to human CD228 protein. Table 8 shows the $EC_{50}$ of the antibodies calculated according to FIG. 3. It can be seen that 6 antibodies exhibited similar ELISA binding activities, which are better than that of the reference antibody HL49.

Table 8. $EC_{50}$ of antibodies

| ID | EC50 (ng/mL) |
|---|---|
| CA13 | 3.2 |

(continued)

| ID | EC50 (ng/mL) |
|---|---|
| CA67 | 3.8 |
| CA149 | 3.6 |
| CA185 | 3.9 |
| BA352 | 6.1 |
| CA518 | 4.3 |
| hL49 | 27.8 |

**4.2 Affinity assay of antibodies for human, monkey, and mouse CD228 proteins**

Table 9. Sources of CD228 proteins for detection

| Name | Lot No. | Manufacturer |
|---|---|---|
| Human CD228 protein (SEQ ID NO: 41) | 20201014 | Shandong Boan Biotechnology Co., Ltd. |
| Mouse CD228 protein (SEQ ID NO: 42) | 20201201 | Shandong Boan Biotechnology Co., Ltd. |
| Monkey CD228 protein (SEQ ID NO: 43) | 20210617 | Shandong Boan Biotechnology Co., Ltd. |

The binding kinetics of the antibodies to the CD228 proteins was measured using the BIAcore 8K system based on surface plasmon resonance (SRP). CD228 antibodies were immobilized on ProA chips at a concentration of 2 μg/mL, and the binding activity of the CD228 antibodies to human, monkey, and mouse CD228 proteins was analyzed. Human, monkey and mouse CD228 proteins were serially 2-fold diluted from 50 nM to 5 concentrations with an HBS-EP+ buffer. The binding kinetics of the CD228 proteins were analyzed by Biacore, and the affinity activity KD values were calculated by fitting.

Table 10. Binding assay of anti-CD228 antibodies to human, monkey, and mouse proteins by Biacore

| Sample | Biacore KD (pM) | | |
|---|---|---|---|
| | Human CD228 | Monkey CD228 | Mouse CD228 |
| hL49 analogy | 4.18E-09 | Low binding | No binding |
| CA13 | 3.45E-08 | 4.89E-08 | No binding |
| CA67 | 3.53E-08 | 4.14E-08 | No binding |
| CA149 | 2.41E-08 | 4.23E-08 | No binding |
| CA185 | 2.48E-09 | 3.81E-09 | No binding |
| BA352 | 1.18E-07 | 1.23E-07 | No binding |
| CA518 | 3.13E-08 | 5.85E-08 | No binding |

As can be seen from Table 10, the antibodies have similar affinity for human CD228 protein and monkey CD228 protein, but do not bind to mouse CD228 protein.

**4.3 Epitope analysis for anti-hCD228 antibodies**

CD228 proteins were immobilized on His chips at 10 μg/mL with a threshold of 0.5 nm. The first CD228 antibody (30 μg/mL) was immobilized, and the competitive binding of the second CD228 antibody (30 μg/mL) was analyzed. The Octet 8K system was used to analyze the response of antibody 2 and to determine whether antibody 1 and antibody 2 were in competition. Table 11 shows Octet antibody epitope response values.

Table 11. Octet antibody epitope response values

| PBST buffer | 0.3472 | 0.3934 | 0.3931 | 0.425 | 0.4093 | 0.4113 | 0.4417 |
|---|---|---|---|---|---|---|---|
| AB# (blank value) | **hl49** | **BA352** | **CA13** | **CA67** | **CA149** | **CA185** | **CA518** |
| **hL49** | 0.0169 | 0.1766 | 0.4179 | 0.4555 | 0.4349 | 0.4318 | 0.4555 |
| **BA352** | 0.0006 | 0.0217 | 0.4245 | 0.4427 | 0.4157 | 0.4256 | 0.4576 |
| **CA13** | 0.3531 | 0.4274 | -0.0011 | -0.0021 | -0.0059 | -0.0061 | -0.0081 |
| **CA67** | 0.3303 | 0.4232 | -0.0065 | -0.007 | -0.012 | -0.0132 | -0.0133 |
| **CA149** | 0.3484 | 0.4422 | 0.0117 | 0.0087 | 0.0039 | 0.0102 | 0.0079 |
| **CA185** | 0.3563 | 0.4586 | 0.0046 | 0.0028 | 0.0089 | 0.0055 | 0.0029 |
| **CA518** | 0.2807 | 0.4146 | 0.006 | 0.0039 | -0.0041 | -0.0043 | -0.0046 |

The final competition analysis was conducted using the following calculation method: 1 - response value/blank value. The results are shown in Table 12, indicating that the epitopes of BA352 and hL49 are similar. The remaining antibodies compete with each other, suggesting similar epitopes (values over 75% indicate epitope correlation).

Table 12. Final competition analysis results

| AB# | **hl49** | **BA352** | **CA13** | **CA67** | **CA149** | **CA185** | **CA518** |
|---|---|---|---|---|---|---|---|
| hL49 | 95% | 55% | -6% | -7% | -6% | -5% | -3% |
| **BA352** | 100% | 94% | -8% | -4% | -2% | -3% | -4% |
| **CA13** | -2% | -9% | 100% | 100% | 101% | 101% | 102% |
| **CA67** | 5% | -8% | 102% | 102% | 103% | 103% | 103% |
| **CA149** | 0% | -12% | 97% | 98% | 99% | 98% | 98% |
| **CA185** | -3% | -17% | 99% | 99% | 98% | 99% | 99% |
| **CA518** | 19% | -5% | 98% | 99% | 101% | 101% | 101% |

**Example 5. Binding activity assay of anti-hCD228 antibodies at cellular level by flow cytometry**

To a 96-well round-bottom plate, human melanoma SK-MEL-5 cells (ATCC, HTB-70) were added with a cell density of 7E4 cells/50 μL/well. The antibodies were serially diluted with an FACS buffer (PBS, Boster Biological Technology, Catalog No. PYG0021) and added to the 96-well round-bottom plate at 50 μL/well. The plate was then incubated at 4 °C for 1 h. The supernatant was discarded after centrifugation at 400 g for 4 min. The plate was washed once with FACS buffer, before a fluorescent secondary antibody (Jackson, 109545-008) at 100 μL/well was added. The mixture was incubated at 4 °C in the dark for 30 min and centrifuged at 400 g for 4 min. The supernatant was discarded. The cells were washed once with FACS buffer, resuspended in FACS buffer at 100 μL/well, and loaded on a flow cytometer (ACEA Pharma, NovoCyte 2060) for analysis. FIG. 4 shows the binding of the anti-CD228 antibodies to human melanoma SK-MEL-5 cells (expressing CD228 proteins). The results suggest that all 6 antibodies exhibit high binding activity to SK-MEL-5 cells and are superior to hL49. The isotype reference antibody in FIG. 4 is an irrelevant antibody targeting other antigens with constant regions similar to the experimental anti-CD228 antibodies but with different variable regions.

**Example 6. Internalization assay of anti-hCD228 monoclonal antibodies**

**1. SK-MEL-5 cells**

To a 96-well round-bottom plate, human melanoma SK-MEL-5 cells diluted with a buffer (PBS, Boster Biological Technology, Cat. No. PYG0021) were added at 5E4 cells/50 μL/well. The antibodies were then diluted with a buffer to a final concentration of 20 μg/mL. The antibodies at 20 μg/mL were added to the round-bottom plate containing 50 μL/well of cells at 50 μL/well. The mixture was incubated for 30 min and centrifuged at 400 g for 4 min to remove the supernatant. After the plate was washed with a pre-cooled buffer twice, a buffer was added at 100 μL/well and the mixture was incubated at 37 °C and 4 °C separately. The reactions were stopped at different time points, and the mixture was centrifuged at 400 g for 4 min to remove the supernatant. Then, a fluorescent secondary antibody (Jackson, 109-545-008) pre-cooled at 4 °C was

added at 100 μL/well, and the mixture was incubated at 4 °C in the dark for 30 min. The cells were washed once with a pre-cooled FACS buffer, then resuspended in FACS buffer at 100 μL/well, and loaded on a flow cytometer (ACEA, NovoCyte 2060) for analysis. FIG. 5A shows the internalization assay results for the anti-CD228 antibodies in human melanoma SK-MEL-5 cells, suggesting that CA13, CA149, and BA352 have higher internalization rates as compared to the reference hL49.

**2. A549-CD228 cells**

Human lung cancer A549-CD228 cells (KYinno, KC-2150) stably expressing exogenous CD228 gene at the logarithmic growth phase were digested. The digestion was terminated with and the cells were diluted in a serum-containing medium. The cells were added to a 96-well round-bottom plate (NEST, Catalog No. 701111) at 1E5 cells/50 μL/well. The antibodies were diluted in a serum-containing medium, and mixed with a labeling reagent (Invitrogen, Z25611) in a molar ratio of 1:3 (with an antibody concentration of 40 nM and a labeling reagent concentration of 120 nM) at room temperature for 5 min. The labeled antibody mixture was added to the cell-containing plate at 50 μL/well. After incubation at 37 °C for 0 h, 2 h, 6 h, and 24 h, the cells were washed once with PBS, then resuspended in PBS at 100 μL/well, and the mean fluorescence intensity (MFI) values were measured on a flow cytometer (ACEA, NovoCyte 2060). The results show that the internalization of CA149 antibody increased over time. FIG. 5B indicates that CA149 exhibits superior internalization activity to the reference antibody hL49.

**Example 7. Antibody-dependent cell-mediated cytotoxicity (effector cells containing luciferase reporter gene)**

An ADCC working solution (an RPMI1640 medium containing 1% FBS) was prepared. Bioassay effector cells (Promega, G7011) were collected and adjusted to a cell density of $2.4\times10^6$ using the ADCC working solution. Target cells SK-MEL-5 (ATCC, HTB-70) were collected and adjusted to a cell density of $8\times10^5$ using the ADCC working solution. The test sample was serially 4-fold diluted from 5 μg/mL to 8 concentrations with the ADCC working solution. The effector cells, target cells, and test sample were added to a white reaction plate (Costar, 3917) at 25 μL, with a total reaction volume of 75 μL, and the reaction system was incubated at 37 °C for 6 h. Bio-Glo Luciferase System (Promega, G7940) was added at 75 μL/well, and after a 15-min reaction, the chemiluminescence value was measured on a microplate reader (BioTek, synergy neo2). FIG. 6 shows the results of ADCC assay for anti-CD228 antibodies in SK-MEL-5 cells, suggesting that the signal was gradually enhanced along with the increase of the concentration of the antibody samples, and that the antibodies have an ADCC effect on SK-MEL-5 cells.

**Example 8. Binding epitope analysis of CA149 antibody to CD228**

Full-length antigen CD228 and an antibody Fab complex (CA149-Fab) were separately prepared. The Cryo-Electron Microscopy Center of Shuimu BioSciences (Hangzhou) Technology Co., Ltd. was entrusted with analysis of the antigen and antibody structures. The study analyzed the amino acid types and side-chain interactions in the three-dimensional model of the epitope. The results show that the CA149 antibody Fab binds to the antigen through 7 hydrogen bonds and 1 salt bridge. The specific interaction sites are shown in Table 13, where the superscript * denotes amino acids in the CA149 Fab light chain, and the non-superscript italicized characters denote amino acids in the CA149 Fab heavy chain.

Table 13. Interaction sites

| | **CD228 amino acid** | **CA149 Fab amino acid** | **Distance (A)** |
|---|---|---|---|
| **Hydrogen bond** | Arg(R)-275 | Asp(D)-49* | 3.23 |
| | Arg(R)-282 | Ser(S)-56* | 2.61 |
| | *Glu(E)-312* | *Thr(T)-28* | *2.59* |
| | *Glu(E)-312* | *Tyr(Y)-32* | *3.52* |
| | *Leu(L)-313* | *Tyr(Y)-32* | *2.6* |
| **Salt bridge** | Arg(R)-275 | Asp(D)-49* | 3.23 |

Additionally, based on the epitope analysis results, the CD228 (with a sequence set forth in SEQ ID NO: 41) antigen prepared by Shandong Boan Biotechnology Co., Ltd. was verified by mutations on specific sites. Single-site mutants hCD228(R275A), hCD228(R282A), hCD228(E312A) and hCD228(L313A), and double-site mutants

hCD228(E312A, L313A), hCD228(R282A, E312A) and hCD228(R275A, R282A) of the hCD228 antigen were constructed and subjected to affinity analysis. The affinity results show that the antigen mutants exhibited reduced binding activity or lost binding activity to the CA149 antibody, indicating that the 4 sites E312A, L313A, R282A, and R275A are key sites for the binding of the antibody to the antigen. The affinity assay results are summarized in Table 14.

Table 14. Affinity assay

| Antibody | Antigen | KD (M) | Response |
|---|---|---|---|
| CA149 | hCD228 | 1.99E-09 | 0.3844 |
| CA149 | hCD228(E312A, L313A) | Reduced affinity | |
| CA149 | hCD228(R282A, E312A) | No binding | |
| CA149 | hCD228(R275A, R282A) | No binding | |
| CA149 | hCD228(R282A) | Reduced affinity | |
| CA149 | hCD228(L313A) | Significantly reduced affinity | |
| CA149 | hCD228(E312A) | No binding | |
| CA149 | hCD228(R275A) | No binding | |

In addition, another variable splicing product, soluble MFI2, i.e., sMFI2, is also present in the human body, and it has been reported that sMFI can pass through the blood-brain barrier via LRP protein (J Neurochem. 2002 Nov;83(4):924-33.doi: 10.1046/j.1471-4159.2002.01201.x.;J Cereb Blood Flow Metab. 2019 Oct;39(10):2074-2088.doi: 10.1177/0271678X18772998). Therefore, to reduce the potential off-target risk, the antibody targeting CD228 should not bind to sMFI2. According to the epitope results and the sequence comparison results of the full-length hCD228 with the soluble antigen sMFI2 (as shown in FIG. 7), it can be seen that the 4 key binding sites described above are not found in the sMFI2 antigen, indicating that CA149 antibody does not bind to sMFI2.

**Example 9. Conjugation study of anti-hCD228 antibody to drug**

Antibody-drug conjugates having the following molecular formula were prepared,

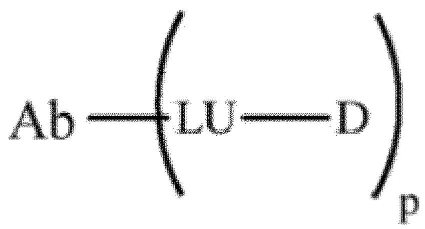

wherein: Ab is any of the anti-CD228 antibodies or an antigen-binding fragment thereof described above, LU is a linker, D is a drug, and the subscript p is the average DAR value of the antibody-drug conjugate.

**9.1 Preparation of anti-hCD228 antibody-vcMMAE antibody-drug conjugates**

The antibody (5-10 mg/mL) in phosphate-buffered saline (pH 7.5, containing 11 mM DTPA) was treated with 2 equivalents of TCEP and then incubated at 25 °C for about 2 h. A solution of vcMMAE (5 equivalents) in DMSO was added to the reduced antibody PBS solution and the mixture was incubated at 25 °C for about 1 h. 10 equivalents of n-acetylcysteine (NAC) was added, and the mixture was incubated at 25 °C for 5 min to quench all unreacted linker-drug. The antibody was subjected to buffer exchange by ultrafiltration to remove free small molecules, and loaded on a HIC-HPLC system for analysis. The analysis results, as shown in Table 15, indicate that the average DAR value of the antibody-drug conjugates used in the present application ranged from 4.01 to 4.42.

Table 15. DC HIC-HPLC results

| Antibody conjugate | DAR 0 | DAR 2 | DAR 4 | DAR 6 | DAR 8 | Average DAR |
|---|---|---|---|---|---|---|
| CA13-vcMMAE | 0.47% | 22.01% | 41.34% | 28.48% | 7.70% | 4.42 |

(continued)

| Antibody conjugate | DAR 0 | DAR 2 | DAR 4 | DAR 6 | DAR 8 | Average DAR |
|---|---|---|---|---|---|---|
| CA67-vcMMAE | 1.25% | 24.90% | 51.33% | 16.95% | 5.57% | 4.01 |
| CA149-vcMMAE | 1.17% | 22.99% | 46.02% | 22.45% | 7.37% | 4.24 |
| CA185-vcMMAE | 1.43% | 25.36% | 47.08% | 19.96% | 6.17% | 4.08 |
| BA352-vcMMAE | 0.48% | 19.85% | 48.65% | 24.55% | 6.47% | 4.33 |
| CA518-vcMMAE | 1.86% | 25.09% | 49.69% | 17.52% | 5.84% | 4.01 |
| hL49-vcMMAE | 0.91% | 23.48% | 49.83% | 19.81% | 5.98% | 4.13 |

### 9.2 Preparation of CA149-BNLD11 antibody-drug conjugate

Taking anti-CD228 antibody CA149 as an example, a homogeneous ADC composition with a drug-to-antibody ratio (DAR) of about 4 was prepared. The antibody (5-10 mg/mL) in phosphate-buffered saline (pH 7.5, containing 11 mM DTPA) was treated with 2 equivalents of TCEP and then incubated at 25 °C for about 2 h. A solution of BNLD-11 (5 equivalents) in DMSO was added to the reduced antibody PBS solution and the mixture was incubated at 25 °C for about 1 h. 10 equivalents of n-acetylcysteine (NAC) was added, and the mixture was incubated at 25 °C for 5 min to quench all unreacted linker-drug. The drug-to-antibody ratio of the antibody-drug conjugate was quantified by HIC-HPLC. The analysis results, as shown in FIG 8 and Table 16, indicate that the average DAR value of the prepared antibody-drug conjugate CA149-BNLD11 in this example is about 4.

Table 16. DAR value integration analysis

| Antibody conjugate | DAR 0 | DAR 1 | DAR 2 | DAR 3 | DAR 4 | DAR 5 | DAR 6 | DAR 7 | DAR 8 | Average DAR |
|---|---|---|---|---|---|---|---|---|---|---|
| CA149-BNLD 11 | 5.4% | 0.0% | 22.0% | 2.3% | 38.9% | 1.9 | 19.9% | 0.77% | 7.84% | 4.1 |

## Example 10. *In vitro* cell killing assay for anti-hCD228 ADCs

SK-MEL-5 cells were added to a 96-well flat-bottom plate (Corning, Cat. No. 3917) containing 10% FBS/EMEM medium at 1E4 cells/50 μL/well. The anti-CD228 ADCs were serially 4-fold diluted from 1 μg/mL to 6 concentrations with the above medium. The diluted ADCs were added to the 96-well flat-bottom plate at 50 μL/well. The plate was then incubated at 37 °C/5% $CO_2$ for 4 days. The CellTiter-Glo kit (Promega, G7571, away from light in use) was equilibrated at room temperature, and the buffer in the kit was mixed well with the substrate and allowed to stand for 1 h. A cell viability detection reagent was added to the 96-well plate at 100 μL/well, before the plate was horizontally shaking at 300 rpm for 2 min and then allowed to stand for 10 min. The mixture was then analyzed on a microplate reader (BioTek, SYNERGY neo, USA).

The cell killing assay results for the anti-CD228 ADCs in human melanoma SK-MEL-5 cells, as shown in FIGs. 9A-9F, indicate that two anti-hCD228 ADCs, CA13-vcMMAE and CA67-vcMMAE, exhibited similar *in vitro* cell killing activities as hL49-vcMMAE; four anti-hCD228 ADCs, CA149-vcMMAE, CA352-vcMMAE, CA518-vcMMAE, and CA185-vcMMAE, exhibited *in vitro* cell killing activities similar with each other but superior to that of reference hL49-vcMMAE; in FIGs. 9A-9F, the isotype reference antibody is an irrelevant antibody targeting other antigens with identical constant regions but with different variable regions as compared with the 6 antibodies of interest.

## Example 11. *In vivo* pharmacodynamic study of anti-CD228 ADCs in mouse xenograft tumor

### 11.1 Pharmacodynamic data in human melanoma SK-MEL-5 cell mouse xenograft tumor model

Human melanoma SK-MEL-5 cells, purchased from ATCC, were cultured in an EMEM medium containing 10% of FBS in an incubator at 37 °C/5% $CO_2$. NOD/SCID mice were purchased from Beijing Vital River Laboratory Animal Technology Co., Ltd. The SK-MEL-5 cells were adjusted to a density of $2.5\times10^7$ cells/mL with an EMEM medium containing 50% of Matrigel and grafted subcutaneously at the right side of NOD/SCID mice at 0.1 mL/mouse. When the mean tumor volume reached about 76 $mm^3$, the mice were divided into 8 groups of 5 mice according to the tumor volume and body weight. The administration was started on the day of grouping, and the dose was 3 mg/kg. FIG 10 shows the PD data (3 mg/kg) of the anti-CD228 ADCs in the SK-MEL-5 animal model, i.e., the changes in tumor volume in mice after administration, indicating that the tumor volume decreased after the administration of the 6 ADCs. FIG. 10 is a graph of the

volume change data in Table 17. As shown in Table 17, the tumor growth inhibition rate (TGI%) was 55.4%, 60.3%, 56.9%, 56.1%, 69.8%, 63.9%, and 49.0% for the CA13-vcMMAE, CA67-vcMMAE, CA149-vcMMAE, CA185-vcMMAE, BA352-Vc-MMAE, CA518-vcMMAE, and hL49-vcMMAE treatment groups, respectively; the tumor weight inhibition rate (IR%) was 59.8%, 65.9%, 61.4%, 55.4%, 68.2%, 65.7%, and 56.1%, respectively. The results indicate that at a dose of 3 mg/kg, all 6 anti-CD228-vcMMAE conjugates prepared in the project show superior tumor growth inhibition rate (TGI%) and tumor weight inhibition rate (IR%) to that of the control group hL49-vcMMAE in the human melanoma SK-MEL-5 mouse xenograft tumor model.

Table 17. Tumor volume changes upon administration at 3 mg/kg

| Groups | Mean volume ($mm^3$) | TGI (%) | IR (%) |
|---|---|---|---|
| Vehicle (PBS, phosphate-buffered saline) | 1027 | \ | \ |
| CA13-vcMMAE | 501 | 55.4 | 59.8 |
| CA67-vcMMAE | 454 | 60.3 | 65.9 |
| CA149-vcMMAE | 486 | 56.9 | 61.4 |
| CA185-vcMMAE | 493 | 56.1 | 55.4 |
| BA352-vcMMAE | 364 | 69.8 | 68.2 |
| CA518-vcMMAE | 420 | 63.9 | 65.7 |
| hL49-vcMMAE | 561 | 49.0 | 56.1 |

FIG. 11 shows the pharmacodynamic data of the anti-CD228 ADCs (5 mg/kg) on the SK-MEL-5 animal model. The NCG mice were purchased from Jiangsu GemPharmatech Co., Ltd. The SK-MEL-5 cells were adjusted to a density of $3\times10^7$ cells/mL with an EMEM medium containing 50% of Matrigel and grafted subcutaneously at the right side of NCG mice at 0.1 mL/mouse. When the mean tumor volume reached about 83 $mm^3$, the mice were divided into 8 groups of 6 mice according to the tumor volume and body weight. The administration was started on the day of grouping, and the dose was 5 mg/kg. The ADCs of interest have better efficacy than that of the reference hL49. FIG 11 is a graph of the volume change data in Table 18. As shown in Table 18, at the end of the study, the tumor growth inhibition (TGI) was 82%, 84%, 81%, 80%, 81%, and 74% for the CA13-vcMMAE, CA149-vcMMAE, CA518-vcMMAE, CA523-vcMMAE, CA579-vcMMAE, and hL49-vcMMAE treatment groups, respectively; the tumor weight inhibition rates were 77.7%, 79.0%, 75.5%, 75.2%, 72.0%, and 69.1%, respectively. The results indicate that at a dose of 5 mg/kg, the anti-CD228-vcMMAE conjugates prepared in the project show superior tumor growth inhibition rate (TGI%) and tumor weight inhibition rate (IR%) to that of the control group hL49-vcMMAE in the human melanoma SK-MEL-5 mouse xenograft tumor model.

Table 18. Tumor volume changes upon administration at 5 mg/kg

| Groups | Mean volume ($mm^3$) | TGI (%) | IR (%) |
|---|---|---|---|
| Vehicle (PBS, phosphate-buffered saline) | 640 | \ | \ |
| CA13-vcMMAE | 184 | 82 | 77.7 |
| CA149-vcMMAE | 171 | 84 | 79.0 |
| CA518-vcMMAE | 191 | 81 | 75.5 |
| CA523-vcMMAE | 193 | 80 | 75.2 |
| CA579-vcMMAE | 190 | 81 | 72.0 |
| CAhL49-vcMMAE | 229 | 74 | 69.1 |

**11.2 Pharmacodynamic data in human NCI-H226 lung cancer cells/mouse xenograft tumor model**

Human lung cancer NCI-H226 cells, purchased from ATCC, were cultured in an RPMI 1640 medium containing 10% of FBS in an incubator at 37 °C/5% $CO_2$. CB-17/SCID mice were purchased from Beijing Vital River Laboratory Animal Technology Co., Ltd.

The NCI-H226 cells were adjusted to a density of $5.0\times10^7$ cells/mL with an RPMI 1640 medium containing 50% of Matrigel and grafted subcutaneously at the right side of CB-17/SCID mice at 0.1 mL/mouse. When the mean tumor volume reached about 156 $mm^3$, the mice were divided into 8 groups of 5 mice according to the tumor volume and body

weight. The administration was started on the day of grouping, and the dose was 3 mg/kg. FIG 12 shows the pharmacodynamic data of anti-CD228 ADCs (3 mg/kg) on the NCI-H226 animal model. It can be seen that the tumor volume decreased after administration of the 6 ADCs, demonstrating similar *in vivo* efficacy as HL49. FIG. 12 is a graph of the volume change data in Table 20. As shown in Table 19, at the end of the study, the tumor growth inhibition (TGI) was 46.7%, 55.2%, 45.3%, 50.5%, 47.7%, 63.4%, and 55.1% for the CA13-vcMMAE, CA67-vcMMAE, CA149-vcMMAE, CA185-vcMMAE, CA352-vcMMAE, CA518-vcMMAE, and hL49-vcMMAE treatment groups, respectively; the tumor weight inhibition rate was 39.7%, 44.5%, 46.9%, 43.1%, 41.6%, 47.8%, and 49.8%, respectively.

Table 19. Tumor volume changes upon administration at 3 mg/kg

| Groups | Mean volume ($mm^3$) | TGI (%) | IR (%) |
|---|---|---|---|
| Vehicle (PBS, phosphate-buffered saline) | 601 | \ | \ |
| CA13-vcMMAE | 393 | 46.7 | 39.7 |
| CA67-vcMMAE | 356 | 55.2 | 44.5 |
| CA149-vcMMAE | 399 | 45.3 | 46.9 |
| CA185-vcMMAE | 376 | 50.5 | 43.1 |
| BA352-vcMMAE | 389 | 47.7 | 41.6 |
| CA518-vcMMAE | 319 | 63.4 | 47.8 |
| hL49-vcMMAE | 356 | 55.1 | 49.8 |

FIG. 13 shows the pharmacodynamic data of the anti-CD228 ADCs (5 mg/kg) on the NCI-H226 animal model. The NCG mice were purchased from Jiangsu GemPharmatech Co., Ltd. The NCI-H226 cells were adjusted to a density of $5\times10^7$ cells/mL with an RPMI 1640 medium containing 50% of Matrigel and grafted subcutaneously at the right side of NCG mice at 0.1 mL/mouse. When the mean tumor volume reached about 108 $mm^3$, the mice were divided into 8 groups of 6 mice according to the tumor volume and body weight. The administration was started on the day of grouping, and the dose was 5 mg/kg. The ADCs of interest have better efficacy than that of the reference HL49. FIG. 13 is a graph of the volume change data in Table 20. As shown in Table 16, at the end of the study, the tumor growth inhibition (TGI) was 94.4%, 92.3%, 94.6%, 98.3%, 103.9%, and 75.6% for the CA13-vcMMAE, CA149-vcMMAE, CA518-vcMMAE, CA523-vcMMAE, CA579-vcMMAE, and hL49-vcMMAE treatment groups, respectively; the tumor weight inhibition rate was 73.1%, 69.2%, 72.1%, 75.5%, 77.4%, and 56.7%, respectively.

Table 20. Tumor volume changes upon administration at 5 mg/kg

| Groups | Mean volume ($mm^3$) | TGI (%) | IR (%) |
|---|---|---|---|
| Vehicle (PBS, phosphate-buffered saline) | 469.8 | \ | \ |
| CA13-vcMMAE | 128.5 | 94.4 | 73.1 |
| CA149-vcMMAE | 136.1 | 92.3 | 69.2 |
| CA518-vcMMAE | 127.7 | 94.6 | 72.1 |
| CA523-vcMMAE | 114.2 | 98.3 | 75.5 |
| CA579-vcMMAE | 93.8 | 103.9 | 77.4 |
| hL49-vcMMAE | 196.1 | 75.6 | 56.7 |

**Example 12. *In vivo* pharmacokinetic study of anti-CD228 ADCs in mice**

Each ADC was administered to 3 Balb/c mice via subcutaneous injection at a dose of 10 mg/kg. The serum antibody concentration was measured by Elisa at the following time points: 0 h pre-dose, and 1 h, 4 h, 10 h, 1 d, 2 d, 3 d, 4 d, 5 d, 7 d, 10 d and 14 d post-dose.

FIG. 14 shows the pharmacokinetic profiles of CA13-vcMMAE, CA67-vcMMAE, and CA149-vcMMAE, and the results shown in FIG. 14 indicate that CA149-vcMMAE exhibited better pharmacokinetic properties than those of CA13-vcMMAE and CA67-vcMMAE constructed on the basis of antibodies CA13 and CA67.

**Example 13. Inhibitory activity of antibody-drug conjugate CA149-BNLD11 against cell proliferation**

**13.1 Inhibitory activity of CA149-BNLD11 against MC38-CD228 cell and A375-CD228 cell proliferation**

MC38-CD228 cells (KYinno, KC-2023) and A375-CD228 cells (KYinno, KC-2110) at the logarithmic growth phase were digested, diluted and resuspended in 10% FBS/1640 medium, and added to a 96-well flat-bottom plate (SARSTED, Cat. No. 94.6120.096) at 1E4 cells/50 μL/well. The antibody-drug conjugate CA149-BNLD11 prepared as described in section 8.2 of Example 8 was serially 5-fold diluted from 60 and 12 μg/mL with a serum-containing medium. The antibody with ID CA521 in Patent No. CN202180003751.7 was used to prepare nCov-CA521-vcMMAE following the same method as in Example 8, and the conjugate was used in the control group (Isotype). The diluted CA149-BNLD11 was added to the 96-well flat-bottom cell culture plate at 50 μL/well. The plate was then incubated at 37 °C/5% $CO_2$ for 96 h. The CellCounting-Lite®2.0 kit (Vazyme, DD1101-01, away from light in use) was equilibrated at room temperature, mixed well by inversion, and added to the 96-well plate at 100 μL/well. The plate was shaken horizontally at 300 rpm for 2 min and then allowed to stand for 10 min. The cell viability was measured on a microplate reader (BioTek, SYNERGY neo, USA).

The results show that CA149-BNLD11 has excellent inhibitory activity against the proliferation of MC38-CD228 and A375-CD228 cells, with the $IC_{50}$ being 101.5 ng/mL and 61.8 ng/mL, respectively, as shown in Table 21. FIGs. 15A and 15B are graphs in which the vertical coordinate represents the percentage of viable cells, i.e., the ratio of viable cells in the treatment group to viable cells in the blank group. FIGs. 15A and 15B indicate that CA149-BNLD11 exhibits significant inhibitory activity against the proliferation of cells expressing CD228 as compared to the control group (Isotype).

Table 21. Data for inhibitory activity of CA149-BNLD11 against proliferation

| **Cell Name** | **MC38-CD228** | **A375-CD228** |
|---|---|---|
| **$IC_{50}$ (ng/mL)** | 101.5 | 61.8 |

**13.2 Inhibitory activity of CA149-BNLD11 against proliferation of SK-MEL-5 cells, A549-CD228 cells, and A375-CD228 cells**

SK-MEL-5 cells (ATCC, HTB-70), A549-CD228 cells (KYinno, KC-2150), and A375-CD228 cells (KYinno, KC-2110) at the logarithmic growth phase were digested. The digestion was terminated with and the cells were diluted in a serum-containing medium. The cells were added to a 96-well flat-bottom plate (SARSTED, Cat. No. 94.6120.096) at 1E4 cells/50 μL/well. CA149-BNLD11 was serially 5-fold, 6-fold, and 5-fold diluted from 1.2, 60, and 6 μ/mL with a serum-containing medium. The diluted ADC was added to the 96-well flat-bottom cell culture plate at 50 μL/well. The plate was then incubated at 37 °C/5% $CO_2$ for 96 h or 120 h. The CellCounting-Lite®2.0 kit (Vazyme, DD1101-01, away from light in use) was equilibrated at room temperature, mixed well by inversion, and added to the 96-well plate at 100 μL/well. The plate was shaken horizontally at 300 rpm for 2 min and then allowed to stand for 10 min. The cell viability was measured on a microplate reader (BioTek, SYNERGY neo, USA). The results show that CA149-BNLD11 has excellent inhibitory activity against the proliferation of SK-MEL-5, A549-CD228, and A375-CD228 cells (with the $IC_{50}$ being 11.48 ng/mL, 15.25 ng/mL and 13.86 ng/mL, respectively). FIGs. 15C-15E indicate that CA149-BNLD11 exhibited significant inhibitory activity against the proliferation of cells expressing CD228 as compared to the control group (Isotype).

Table 22. Data for inhibitory activity of CA149-BNLD11 against proliferation of cells expressing CD228

| **Cell Name** | SK-MEL-5 | A549-CD228 | A375-CD228 |
|---|---|---|---|
| **$IC_{50}$ (ng/mL)** | 11.48 ng/mL | 15.25 ng/mL | 13.86 ng/mL |

**Example 14. Efficacy assessment of antibody-drug conjugate CA149-BNLD11 in human lung cancer cell CaLu-1 nude mice xenograft tumor**

CaLu-1 human lung cancer cells, purchased from ATCC, were cultured in McCoy's 5A medium containing 10% of FBS in an incubator at 37 °C/5% $CO_2$. Balb/c nude mice were purchased from Jiangsu GemPharmatech Co., Ltd. The CaLu-1 cells were adjusted to a density of $5.0\times10^7$ cells/mL with a serum-free McCoy's 5A medium containing 50% Matrigel and grafted subcutaneously at the right side of Balb/c nude mice at 0.1 mL/mouse. When the mean tumor volume reached about 135 $mm^3$, the mice were divided into 5 groups of 5 mice according to the tumor volume. The administration was started on the day of grouping. A single dose was given to the mice, and the mice were observed for 28 days after administration.

The results are recorded in FIGs. 16 and 17. FIG 16 shows the tumor growth inhibition curves of the antibody-drug

conjugate treatment groups in the Calu-1 lung cancer model, and FIG. 17 shows the tumor weight column chart of the antibody-drug conjugate treatment group in the Calu-1 lung cancer model.

As shown in FIG. 16, compared to the vehicle control group (PBS, phosphate-buffered saline), all antibody-drug conjugate treatment groups exhibited significantly inhibited tumor volume growth ($P < 0.05$). CA149-BNLD11, CA149-vcMMAE, and CA149-GGFG-Dxd treatment groups exhibited significantly better tumor inhibition effects as compared to the nCov-CA521-vccMMAE group (with P values being 0.0001, 0.0016 and 0.0006, respectively). There were no significant differences among the CA149-BNLD11, CA149-vcMMAE, and CA149-GGFG-Dxd groups ($P > 0.05$). The tumor growth inhibition (TGI%) was 103.1%, 93.9%, 97.3% and 48.5% for the CA149-BNLD11, CA149-vcMMAE, CA149-GGFG-Dxd, and nCov-CA521-vcMMAE treatment groups, respectively.

As shown in FIG 17, at the end of the study, the mean tumor weights of the CA149-BNLD11, CA149-vcMMAE, and CA149-GGFG-Dxd treatment groups were significantly lower than that of the vehicle control group (PBS, phosphate-buffered saline) ($P < 0.05$). The tumor weights of the nCov-CA521-vcMMAE treatment group and the control group exhibited no statistical differences ($P = 0.3250$). There were no significant differences among the three treatment groups of CA149-BNLD11, CA149-vcMMAE, and CA149-GGFG-Dxd ($P > 0.05$). The tumor weight inhibition rate was 96.3%, 87.3%, 87.6%, and 44.3% for the CA149-BNLD11, CA149-vcMMAE, CA149-GGFG-Dxd, and nCov-CA521-vcMMAE treatment groups, respectively.

During the study, all animals demonstrated good activity and food intake and certain weight gain, indicating that the drug was well tolerated in the animals. No significant differences were observed among the groups ($P > 0.05$).

**Example 15. Efficacy assessment of antibody-drug conjugate CA149-BNLD11 in human melanoma cell SK-MEL-5 nude mice xenograft tumor**

SK-MEL-5 human melanoma cells, purchased from ATCC, were cultured in an EMEM medium containing 10% of FBS in an incubator at 37 °C/5% $CO_2$. Balb/c nude mice were purchased from Jiangsu GemPharmatech Co., Ltd. The SK-MEL-5 cells were adjusted to a density of $5.0\times10^7$ cells/mL with a serum-free EMEM medium containing 50% of Matrigel and grafted subcutaneously at the right side of Balb/c nude mice at 0.1 mL/mouse. When the mean tumor volume reached about 98 $mm^3$, the mice were divided into 4 groups of 5 mice according to the tumor volume. The administration was started on the day of grouping. A single dose was given to the mice, and the mice were observed for 28 days after administration.

The results are recorded in FIGs. 18 and 19. FIG. 18 shows the tumor growth inhibition curves of the antibody-drug conjugate groups in the human melanoma cell SK-MEL-5 nude mice xenograft tumor model, and FIG. 19 shows the tumor weight column chart of the antibody-drug conjugate groups in the human melanoma cell SK-MEL-5 nude mice xenograft tumor model. As shown in tumor growth curves in FIG 18, compared to the vehicle control group (PBS, phosphate-buffered saline), all treatment groups exhibited significantly inhibited tumor volume growth ($P < 0.0001$). CA149-BNLD11, CA149-vcMMAE, and CA149-GGFG-Dxd treatment groups exhibited significantly better tumor inhibition effects as compared to the nCov-CA521-vcMMAE group ($P < 0.0001$), and complete tumor regression was observed in 2/5 mice in the CA149-BNLD11 treatment group. There were no significant differences among the CA149-BNLD11, CA149-vcMMAE, and CA149-GGFG-Dxd groups ($P > 0.05$). The tumor growth inhibition (TGI%) was 95.4%, 99.5%, 99.2% and 58.3% for the CA149-BNLD11, CA149-vcMMAE, CA149-GGFG-Dxd, and nCov-CA521-vcMMAE treatment groups, respectively.

As shown in FIG 19, at the end of the study, the mean tumor weights of all treatment groups were significantly lower than that of the vehicle control group (PBS, phosphate-buffered saline) ($P < 0.05$). There were no significant differences among the four treatment groups of CA149-BNLD11, CA149-vcMMAE, CA149-GGFG-Dxd, and nCov-CA521-vcMMAE ($P > 0.05$). The tumor weight inhibition rate was 88.3%, 90.7%, 90.5%, and 58.7% for the CA149-BNLD11, CA149-vcMMAE, CA149-GGFG-Dxd, and nCov-CA521-vcMMAE treatment groups, respectively.

During the study, all animals demonstrated good activity and food intake, and groups other than the vehicle control group (PBS, phosphate-buffered saline) exhibited certain weight gain, indicating that the drug was well tolerated in the animals. No significant differences were observed among the groups ($P > 0.05$).

**Example 16. Efficacy assessment of antibody-drug conjugate CA149-BNLD11 in human gastric cancer cell NUGC4 Balb/c nude mice xenograft tumor**

NUGC4 human gastric cancer cells, purchased from Kyinno Biotechnology (Beijing) Co., Ltd.Kyinno Biotechnology (Beijing) Co., Ltd., were cultured in RMPI-1640 medium containing 10% of FBS in an incubator at 37 °C/5% $CO_2$. Balb/c nude mice were purchased from Jiangsu GemPharmatech Co., Ltd. The NUGC4 cells were adjusted to a density of $1.8\times10^7$ cells/mL with a serum-free RMPI-1640 medium containing 50% of Matrigel and grafted subcutaneously at the right side of Balb/c nude mice at 0.1 mL/mouse. When the mean tumor volume reached about 108 $mm^3$, the mice were divided into 4 groups of 5 mice according to the tumor volume. The administration was started on the day of grouping. A single dose was given to the mice, and the mice were observed for 23 days after administration.

The results are recorded in FIGs. 20 and 21. FIG 20 shows the tumor growth inhibition curves of the antibody-drug conjugate groups in the human gastric cancer cell NUGC4 Balb/c nude mice xenograft tumor model, and FIG. 21 shows the tumor weight column chart of the antibody-drug conjugate groups in the human gastric cancer cell NUGC4 Balb/c nude mice xenograft tumor model.

As shown in tumor growth curves in FIG. 20, at the end of the study, compared to the vehicle control group (PBS, phosphate-buffered saline), the CA149-BNLD11, CA149-vcMMAE, and CA149-GGFG-Dxd treatment groups exhibited significantly inhibited tumor volume growth ($P < 0.0001$). CA149-BNLD11, CA149-vcMMAE, and CA149-GGFG-Dxd treatment groups exhibited significantly better tumor inhibition effects as compared to the nCov-CA521-vcMMAE group ($P < 0.0001$), and the CA149-BNLD11 treatment group exhibited a significantly better tumor inhibitory effect than that of the CA149-GGFG-Dxd treatment group ($P = 0.0225$). There were no significant differences between the CA149-vcMMAE and CA149-GGFG-Dxd treatment groups ($P = 0.1902$). The tumor growth inhibition (TGI%) was 104.2%, 97.7%, 81.0% and 23.0% for the CA149-BNLD11, CA149-vcMMAE, CA149-GGFG-Dxd, and nCov-CA521-vcMMAE treatment groups, respectively.

As shown in FIG 21, at the end of the study, the mean tumor weights of the CA149-BNLD11, CA149-vcMMAE, and CA149-GGFG-Dxd treatment groups were significantly lower than that of the vehicle control group (PBS, phosphate-buffered saline) ($P \leq 0.0001$). The tumor weights of the nCov-CA521-vcMMAE treatment group and the control group exhibited no statistical differences ($P = 0.8144$). The tumor weights of the CA149-BNLD11, CA149-vcMMAE, and CA149-GGFG-Dxd treatment groups were significantly lower than that of the nCov-CA521-vcMMAE group ($P > 0.05$). The tumor weight inhibition rate was 95.7%, 87.1%, 79.4%, and 14.8% for the CA149-BNLD11, CA149-vcMMAE, CA149-GGFG-Dxd, and nCov-CA521-vcMMAE treatment groups, respectively.

During the study, all animals demonstrated good activity and food intake and certain weight gain, indicating that the drug was well tolerated in the animals. No significant differences were observed among the groups ($P > 0.05$).

### Example 17. Efficacy assessment of antibody-drug conjugate CA149-BNLD11 in human squamous cell lung cancer cell NCI-H226 Balb/c nude mice xenograft tumor

NCI-H226 human squamous cell lung cancer cells, purchased from ATCC, were cultured in an RMPI-1640 medium containing 10% of FBS in an incubator at 37 °C/5% $CO_2$. Balb/c nude mice were purchased from Jiangsu GemPharmatech Co., Ltd. The NCI-H226 cells were adjusted to a density of $4.0\times10^7$ cells/mL with a serum-free RMPI-1640 medium containing 50% of Matrigel and grafted subcutaneously at the right side of Balb/c nude mice at 0.1 mL/mouse. When the mean tumor volume reached about 145 $mm^3$, the mice were divided into 4 groups of 6 mice according to the tumor volume. The administration was started on the day of grouping. The treatment was given in a single dose at 3.3 mg/kg. The results were expressed in Mean $\pm$ SEM, and the data were analyzed and processed using Graphpad 8.0 software. The tumor volume and body weight were compared between groups at each time point using a two-way analysis of variance. Statistical differences in tumor weight were analyzed using a one-way analysis of variance. Comparisons between the two groups were conducted using T-tests. $P < 0.05$ indicates significant differences.

The study ended on day 24 after grouping and administration. As shown in the tumor volume growth curves of human squamous cell lung cancer cell NCI-H226 Balb/c nude mice xenograft tumor in FIG. 22: compared to the vehicle control group (PBS, phosphate-buffered saline), CA149-vcMMAE, CA149-BNLD11, and CA149-GGFG-Dxd treatment groups exhibited significantly inhibited tumor volume growth ($P < 0.05$). The nCov-CA521-vcMMAE group exhibited no tumor inhibition effects ($P4 > 0.05$). There were no significant differences among the CA149-vcMMAE, CA149-BNLD11, and CA149-GGFG-Dxd groups ($P > 0.05$). The tumor growth inhibition (TGI%) was 86.6%, 81.1%, 93.5%, and 19.1% for the CA149-vcMMAE, CA149-BNLD11, CA149-GGFG-Dxd, and nCov-CA521-vcMMAE treatment groups, respectively.

### Example 18. Efficacy assessment of multiple doses of antibody-drug conjugate CA149-BNLD11 in human melanoma cell SK-MEL-5 Balb/c nude mice xenograft tumor

SK-MEL-5 human melanoma cells, purchased from ATCC, were cultured in an EMEM medium containing 10% of FBS in an incubator at 37 °C/5% $CO_2$. Balb/c nude mice were purchased from Jiangsu GemPharmatech Co., Ltd. The SK-MEL-5 cells were adjusted to a density of $3.0\times10^7$ cells/mL with a serum-free EMEM medium containing 50% of Matrigel and grafted subcutaneously at the right side of Balb/c nude mice at 0.1 mL/mouse. When the mean tumor volume reached about 103 $mm^3$, the mice were divided into 4 groups of 6 mice according to the tumor volume. The administration was started on the day of grouping. The treatment was given in a single dose at 1.0 mg/kg, 2.5 mg/kg, or 5.0 mg/kg. The results were expressed in Mean $\pm$ SEM, and the data were analyzed and processed using Graphpad 8.0 software. The tumor volume and body weight were compared between groups at each time point using a two-way analysis of variance. Statistical differences in tumor weight were analyzed using a one-way analysis of variance. Comparisons between the two groups were conducted using T-tests. $P < 0.05$ indicates significant differences.

The study ended on day 21 after grouping and administration. As shown in the tumor volume growth curves of

human melanoma cell SK-MEL-5 Balb/c nude mice xenograft tumor in FIG 23: compared to the vehicle control group (PBS, phosphate-buffered saline), the 2.5 mg/kg and 5.0 mg/kg treatment groups exhibited significantly inhibited tumor volume growth ($P < 0.05$). There were no significant differences between the 1.0 mg/kg group and the vehicle control group (PBS, phosphate-buffered saline) ($P = 0.4030$). The tumor growth inhibition (TGI%) was 25.1%, 72.1%, and 91.1% for the CA149-BNLD11 1.0 mg/kg, 2.5 mg/kg, and 5.0 mg/kg treatment groups, respectively. The results indicate a dose-dependence in the tumor inhibitory activity of CA149-BNLD 11.

As shown in the tumor weight growth curves of human melanoma cell SK-MEL-5 Balb/c nude mice xenograft tumor in FIG 24: at the end of the study, compared to the vehicle control group (PBS, phosphate-buffered saline), the tumor weights were significantly reduced in the 2.5 mg/kg and 5.0 mg/kg groups ($P < 0.05$). There were no significant differences between the 1.0 mg/kg group and the vehicle control group (PBS, phosphate-buffered saline) ($P = 0.7086$). The tumor weight inhibition rate was 21.9%, 66.7%, and 76.6% for the CA149-BNLD11 1.0 mg/kg, 2.5 mg/kg, and 5.0 mg/kg treatment groups, respectively.

During the study, all animals demonstrated good activity and food intake and certain weight gain, indicating that the drug was well tolerated in the animals. No significant differences were observed among the groups ($P > 0.05$).

**Example 19. Metabolic study of antibody-drug conjugate CA149-BNLD11 in mice**

3 ICR mice received CA149-BNLD11 and CA149-vcMMAE via tail vein injection at 10 mg/kg. Serum samples were collected pre-dose, and at 1 h, 6 h, 24 h, 3 d, 5 d, 7 d, 10 d, 14 d, 21 d and 28 d post-dose. The serum antibody concentration was determined by ELISA. The results are detailed in the table below.

Table 23. Key metabolic parameters of CA149-BNLD11 and CA149-vcMMAE in ICRmice

| | CA149-BNLD11-Total Ab | | CA149-BNLD 11-ADC | | CA149-vcMMAE-Total Ab | | CA149-vcMMAE-Total ADC | |
|---|---|---|---|---|---|---|---|---|
| | **mean** | **SD** | **mean** | **SD** | mean | SD | mean | SD |
| $T_{1/2}$ (hour) | 311.20 | 50.00 | 181.70 | 12.14 | 261.23 | 8.47 | 125.50 | 19.56 |
| $T_{max}$ (hour) | 0.08 | 0.00 | 0.08 | 0.00 | 0.39 | 0.53 | 0.39 | 0.53 |
| $C_{max}$ (μg/mL) | 225.09 | 17.81 | 278.02 | 16.99 | 198.99 | 24.37 | 208.24 | 26.33 |
| C0 (μg/mL) | 234.32 | 17.46 | 286.22 | 19.03 | 197.52 | 32.89 | 212.34 | 31.17 |
| $AUC_{0-t}$ (hour*μg/mL) | 17936.17 | 665.99 | 14818.84 | 783.12 | 12508.61 | 360.82 | 6464.14 | 434.92 |
| $AUC_{0-\omega}$ (hour*μg/mL) | 22238.47 | 1954.64 | 15727.84 | 986.66 | 14056.33 | 559.41 | 6512.34 | 415.10 |
| $V_z$ (mL/kg) | 201.03 | 14.73 | 166.65 | 2.89 | 268.59 | 18.38 | 280.25 | 59.81 |
| Cl (mL/hour/kg) | 0.45 | 0.04 | 0.64 | 0.04 | 0.71 | 0.03 | 1.54 | 0.10 |
| $MRT_{0-t}$ (hour) | 391.46 | 56.19 | 201.36 | 14.49 | 266.56 | 25.92 | 90.22 | 13.71 |

As shown in Table 23 and FIG. 25, the *in vivo* metabolic study in mice indicates that CA149-BNLD11 has a longer half-life than that of CA149-vcMMAE. The total antibody exposure of CA149-BNLD11 was 1.4 folds that of CA149-vcMMAE, and the total ADC exposure of CA149-BNLD11 was 2.3 folds that of CA149-vcMMAE. Additionally, the metabolic curves (FIG. 25) show that the drop-off rate of BNLD11 in mice is much lower than that of mc-vcMMAE. In conclusion, the CA149-BNLD11 metabolism is more stable in mice.

**Example 20. Toxicity study of antibody-drug conjugate CA149-BNLD11 in mice**

Balb/c mice were purchased from Jinan Pengyue Laboratory Animal Breeding Co., Ltd. Mice were divided into 6 groups of 3 mice according to the gender and body weight. On the day of grouping, the body weight and food consumption were determined, and the treatment began two days later. The dosing regimen is shown in Table 24.

Table 24. *In vivo* dosing regimen in mice

| Group | Gender | Number/group | Dose | Administration volume | Route and frequency of administration | Observation period, frequency, and indexes |
|---|---|---|---|---|---|---|
| Vehicle (PBS, phosphate-buffered saline) | Female♀ | 3 | \ | 10 mL/kg | Tail vein, single dose | The weight, food consumption, activity, mental state, hair, etc.; thrice a week; |
| CA149-vcMMAE | | | 50 mg/kg | | | |
| CA149-BNLD11 | | | 50 mg/kg | | | |
| Vehicle (PBS, phosphate-buffered saline) | Male♂ | 3 | \ | 10 mL/kg | Tail vein, single dose | The weight, food consumption, activity, mental state, hair, etc.; thrice a week; |
| CA149-vcMMAE | | | 50 mg/kg | | | |
| CA149-BNLD11 | | | 50 mg/kg | | | |

The study ended on day 14 after administration. The results were expressed in Mean ± SEM, and the data were analyzed and processed using Graphpad 8.0 software. The body weight and food consumption were compared using T-tests. $P < 0.05$ indicates significant differences.

As shown in FIGs. 26 and 27, the body weights of mice were significantly reduced by CA149-vcMMAE of CA149-BNLD11 at 50 mg/kg, as compared to the vehicle control group (PBS, phosphate-buffered saline). The maximum weight loss was observed on day 4 after administration. The maximum weight loss in females was 17.44% and 10.04%, and in males, it was 17.97% and 5.24%. The mice in the CA149-vcMMAE group all exhibited piloerection and lethargy to different extents on days 4 and 6 after administration, which returned to normal on day 8 after the administration. In contrast, the mice in the CA149-BNLD11 group were normal. On days 4 and 6 after administration, the toxicity of CA149-vcMMAE was significantly higher than that of CA149-BNLD11 (female: $P < 0.05$; male: $P < 0.05$). Compared to the vehicle control group (PBS, phosphate-buffered saline), the food consumption of mice was significantly reduced by CA149-vcMMAE of CA149-BNLD11 at 50 mg/kg. No significant differences between the CA149-vcMMAE and CA149-BNLD11 groups were observed.

**Example 21. Pre-toxicology study and toxicokinetic study of antibody-drug conjugate CA149-BNLD11 in cynomolgus monkey**

4 cynomolgus monkeys, two males and two females, were selected. At the start of treatment, the body weights of the male monkeys were 3.1 kg and 3.7 kg, and the body weights of the female monkeys were 2.9 kg and 3.7 kg. The monkeys were divided into 3 groups: CA149-BNLD11 low-dose group (2 mg/kg), CA149-BNLD11 medium-dose group (6 mg/kg), and CA149-BNLD11 high-dose group (10 mg/kg). The CA149-BNLD11 low-dose group (2 mg/kg) included one male and one female, the CA149-BNLD11 medium-dose group (6 mg/kg) included one male, and the CA149-BNLD11 high-dose group (10 mg/kg) included one female. The administration volume was 5 mg/mL, with corresponding administration concentrations being 0.4, 1.2, and 2 mg/mL, respectively. Two doses were given to the low-dose and high-dose groups, while the medium-dose group received a single dose via a 30-min intravenous infusion.

After the administration, in addition to the clinical observations, food consumption and body weight monitoring, hematological and biochemistry tests, and toxicokinetic tests were conducted. Blood was collected post-dose, and 0.5 h, 2 h, 6 h, 24 h, 72 h, 120 h, 168 h, 240 h, 336 h, and 504 h after the start of administration for the medium-dose (6 mg/kg) group and for the first dose of the high-dose (10 mg/kg) group.

During the study, no animal experienced life-threatening events or death, and no abnormalities were observed in

any dose group. The body weight exhibited mild changes in all groups, with no drug-related abnormalities observed. The food consumption exhibited irregular changes in all groups, with no drug-related abnormalities observed. As shown in FIG. 28: compared to the indicators before administration, CA149-BNLD11 caused a decrease in hematological indicators WBC, #NEUT, and %NEUT in cynomolgus monkeys 5-14 days after administration at 6 mg/kg or a higher dose, with a recovery trend observed 21 days after the administration. In addition, other hematological indicators in the animals in all groups were basically within the normal range at all measurement time points, with no dose-response or time-response changes. No drug-related abnormalities were observed. Compared to the level pre-dose, CA149-BNLD11 caused an increasing trend of the serum AST level in cynomolgus monkeys in the 10 mg/kg group 5 days after the treatment, which returned to normal 8 days after the treatment. In addition, other biochemistry indicators in the animals in all groups were basically within the normal range at all measurement time points, with no drug-related abnormalities observed.

As shown in FIG. 29, the results of the toxicokinetic study show that the toxin drop-off rate in the 10 mg/kg dose group was significantly lower than that in the 2 mg/kg dose group. The toxin drop-off rate in the 10 mg/kg dose group was similar to that in the 6 mg/kg dose group.

## Claims

**1.** An anti-CD228 antibody or an antigen-binding fragment thereof, comprising 3 light chain complementarity determining regions and 3 heavy chain complementarity determining regions, wherein

the 3 light chain complementarity determining regions of the antibody or the antigen-binding fragment thereof comprise an LCDR1 set forth in SEQ ID NO: 13, an LCDR2 set forth in SEQ ID NO: 14, and an LCDR3 set forth in SEQ ID NO: 15, and the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 18;

the 3 light chain complementarity determining regions of the antibody or the antigen-binding fragment thereof comprise an LCDR1 set forth in SEQ ID NO: 19, an LCDR2 set forth in SEQ ID NO: 20, and an LCDR3 set forth in SEQ ID NO: 21, and the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 22;

the 3 light chain complementarity determining regions of the antibody or the antigen-binding fragment thereof comprise an LCDR1 set forth in SEQ ID NO: 23, an LCDR2 set forth in SEQ ID NO: 20, and an LCDR3 set forth in SEQ ID NO: 21, and the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 22;

the 3 light chain complementarity determining regions of the antibody or the antigen-binding fragment thereof comprise an LCDR1 set forth in SEQ ID NO: 24, an LCDR2 set forth in SEQ ID NO: 25, and an LCDR3 set forth in SEQ ID NO: 26, and the 3 heavy chain complementarity determining regions of the antibody or the antigen-binding fragment thereof comprise an HCDR1 set forth in SEQ ID NO: 16, an HCDR2 set forth in SEQ ID NO: 27, and an HCDR3 set forth in SEQ ID NO: 28;

the 3 light chain complementarity determining regions of the antibody or the antigen-binding fragment thereof comprise an LCDR1 set forth in SEQ ID NO: 29, an LCDR2 set forth in SEQ ID NO: 25, and an LCDR3 set forth in SEQ ID NO: 30, and the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 28;

the 3 light chain complementarity determining regions of the antibody or the antigen-binding fragment thereof comprise an LCDR1 set forth in SEQ ID NO: 31, an LCDR2 set forth in SEQ ID NO: 14, and an LCDR3 set forth in SEQ ID NO: 21, and the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 32;

the 3 light chain complementarity determining regions of the antibody or the antigen-binding fragment thereof comprise an LCDR1 set forth in SEQ ID NO: 19, an LCDR2 set forth in SEQ ID NO: 44, and an LCDR3 set forth in SEQ ID NO: 45, and the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 49; or,

the 3 light chain complementarity determining regions of the antibody or the antigen-binding fragment thereof comprise an LCDR1 set forth in SEQ ID NO: 46, an LCDR2 set forth in SEQ ID NO: 47, and an LCDR3 set forth in SEQ ID NO: 48, and the 3 heavy chain complementarity determining regions of the antibody or the antigen-binding fragment thereof comprise an HCDR1 set forth in SEQ ID NO: 16, an HCDR2 set forth in SEQ ID NO: 17, and an HCDR3 set forth in SEQ ID NO: 28.

**2.** The antibody or the antigen-binding fragment thereof according to claim 1, wherein

the antibody or the antigen-binding fragment thereof comprises a light chain variable region set forth in SEQ ID NO: 1 and a heavy chain variable region set forth in SEQ ID NO: 2;
the antibody or the antigen-binding fragment thereof comprises a light chain variable region set forth in SEQ ID NO: 3 and a heavy chain variable region set forth in SEQ ID NO: 4;
the antibody or the antigen-binding fragment thereof comprises a light chain variable region set forth in SEQ ID NO: 5 and a heavy chain variable region set forth in SEQ ID NO: 6;
the antibody or the antigen-binding fragment thereof comprises a light chain variable region set forth in SEQ ID NO: 7 and a heavy chain variable region set forth in SEQ ID NO: 8;
the antibody or the antigen-binding fragment thereof comprises a light chain variable region set forth in SEQ ID NO: 9 and a heavy chain variable region set forth in SEQ ID NO: 10;
the antibody or the antigen-binding fragment thereof comprises a light chain variable region set forth in SEQ ID NO: 11 and a heavy chain variable region set forth in SEQ ID NO: 12;
the antibody or the antigen-binding fragment thereof comprises a light chain variable region set forth in SEQ ID NO: 38 and a heavy chain variable region set forth in SEQ ID NO: 37; or
the antibody or the antigen-binding fragment thereof comprises a light chain variable region set forth in SEQ ID NO: 40 and a heavy chain variable region set forth in SEQ ID NO: 39.

**3.** The antibody or the antigen-binding fragment thereof according to claim 1 or 2, wherein the antibody comprises a heavy chain constant region set forth in SEQ ID NO: 33 and/or a light chain constant region set forth in SEQ ID NO: 34.

**4.** A nucleic acid, encoding the anti-CD228 antibody or the antigen-binding fragment thereof according to any one of claims 1-3.

**5.** A cell, comprising the nucleic acid according to claim 4.

**6.** An antibody-drug conjugate (ADC), wherein the ADC has a structure represented by formula 1 below:

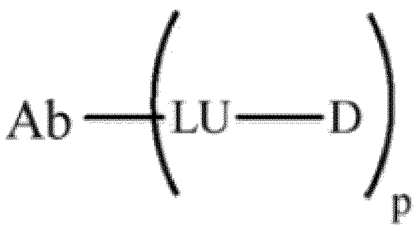

------------Formula 1

Ab is the anti-CD228 antibody or the antigen-binding fragment thereof according to any one of claims 1-3;
LU is a linker;
D is a drug;
p corresponds to the average DAR value for the antibody-drug conjugate, and is a value selected from 1-10, preferably 1-8, more preferably 1-4 or 4-8, and even more preferably 4.

**7.** The antibody-drug conjugate according to claim 6, wherein the LU-D structure is VcMMAE, Vc is valine-citrulline, and MMAE is monomethyl auristatin E.

**8.** The antibody-drug conjugate according to claim 6, wherein the LU-D structure is the structure shown below:

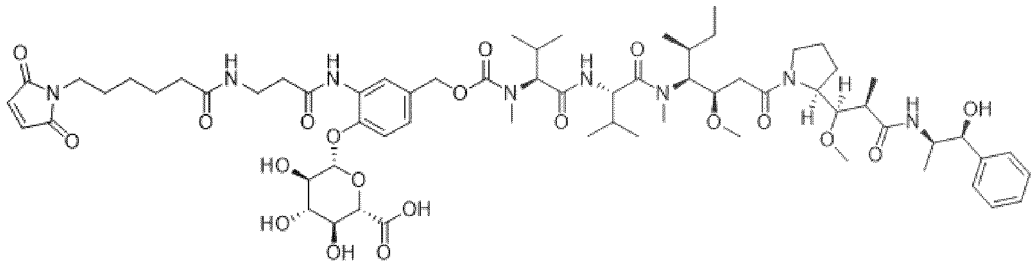

9. The antibody-drug conjugate according to claim 7 or 8, wherein the 3 light chain complementarity determining regions of the antibody or the antigen-binding fragment thereof comprise an LCDR1 set forth in SEQ ID NO: 24, an LCDR2 set forth in SEQ ID NO: 25, and an LCDR3 set forth in SEQ ID NO: 26, and the 3 heavy chain complementarity determining regions of the antibody or the antigen-binding fragment thereof comprise an HCDR1 set forth in SEQ ID NO: 16, an HCDR2 set forth in SEQ ID NO: 27, and an HCDR3 set forth in SEQ ID NO: 28;

   preferably, the antibody or the antigen-binding fragment thereof comprises a light chain variable region set forth in SEQ ID NO: 7 and a heavy chain variable region set forth in SEQ ID NO: 8;
   more preferably, the heavy chain constant region sequence of the antibody or the antigen-binding fragment thereof is set forth in SEQ ID NO 33 and/or the light chain constant region sequence of the antibody or the antigen-binding fragment thereof is set forth in SEQ ID NO 34.

10. A pharmaceutical composition, comprising the anti-CD228 antibody or the antigen-binding fragment thereof according to any one of claims 1-3, the nucleic acid according to claim 4, the cell according to claim 5, or the antibody-drug conjugate according to any one of claims 6-9.

11. A kit, comprising the anti-CD228 antibody or the antigen-binding fragment thereof according to any one of claims 1 to 3, the nucleic acid according to claim 4, the antibody-drug conjugate according to any one of claims 6 to 9, or the pharmaceutical composition according to claim 10.

12. Use of the anti-CD228 antibody or the antigen-binding fragment thereof according to any one of claims 1-3, the nucleic acid according to claim 4, the antibody-drug conjugate according to any one of claims 6-9, the pharmaceutical composition according to claim 10, or the kit according to claim 11 in preparing a reagent for preventing, treating, detecting, or diagnosing a CD228-associated disease, wherein preferably, the CD228-associated disease includes one or more of melanoma, lung cancer, gastric cancer, colon cancer, mesothelioma, pancreatic cancer, and breast cancer.

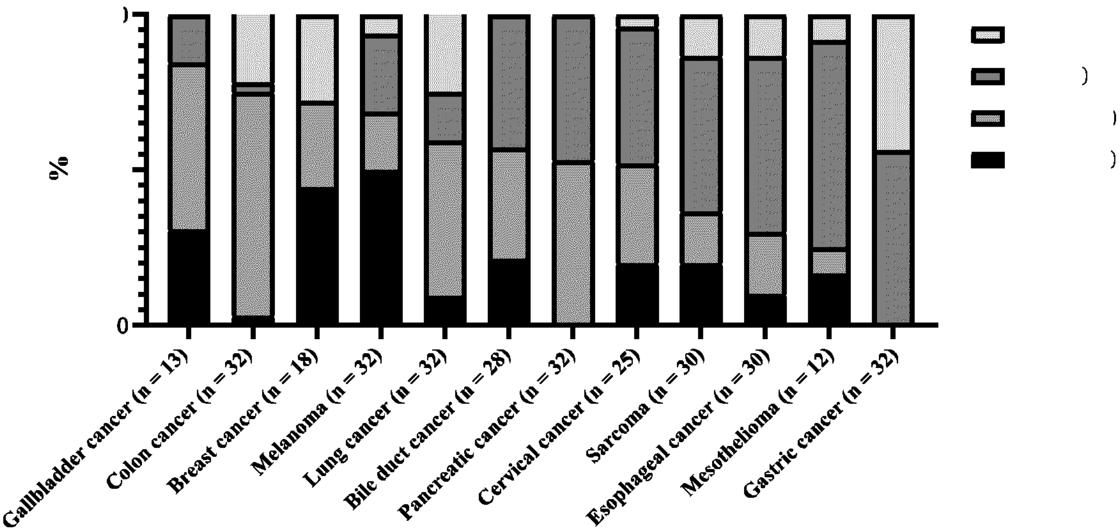


FIG. 1

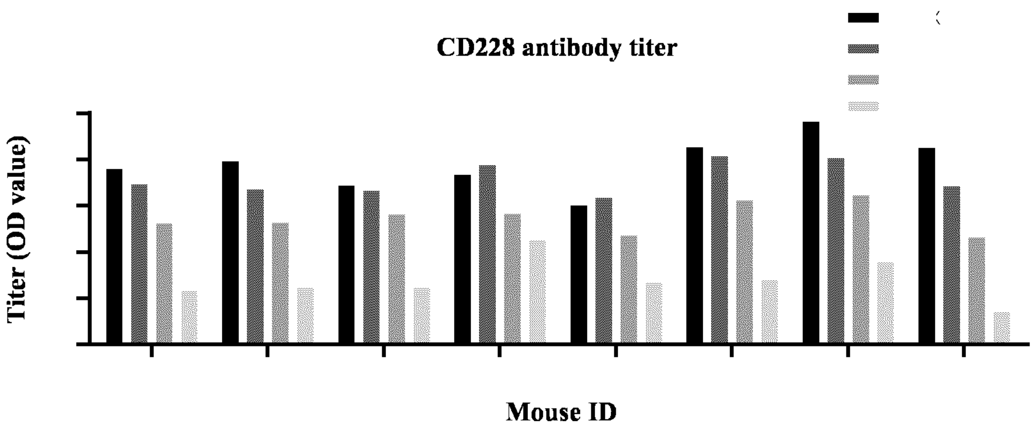


FIG. 2

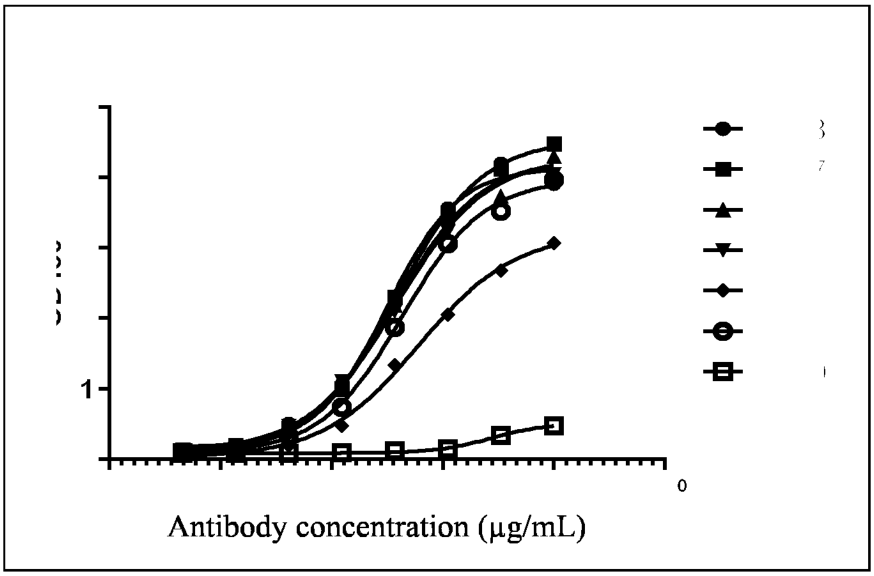


FIG. 3

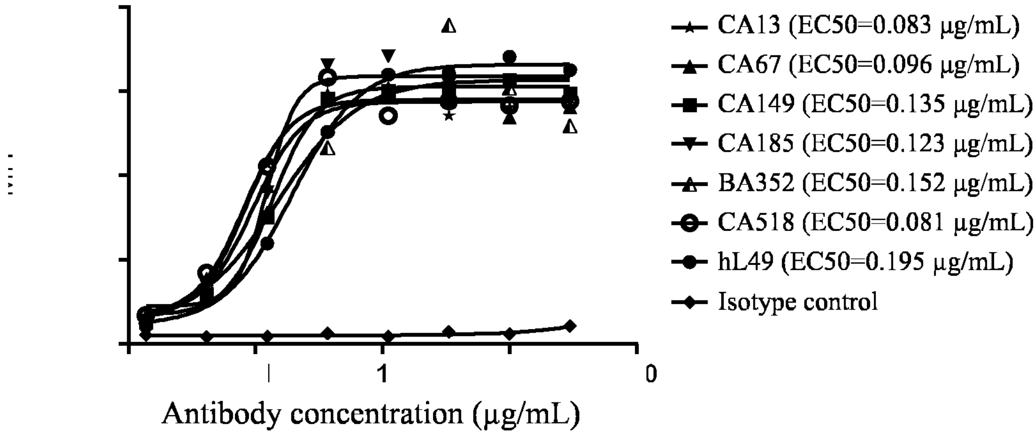


FIG. 4

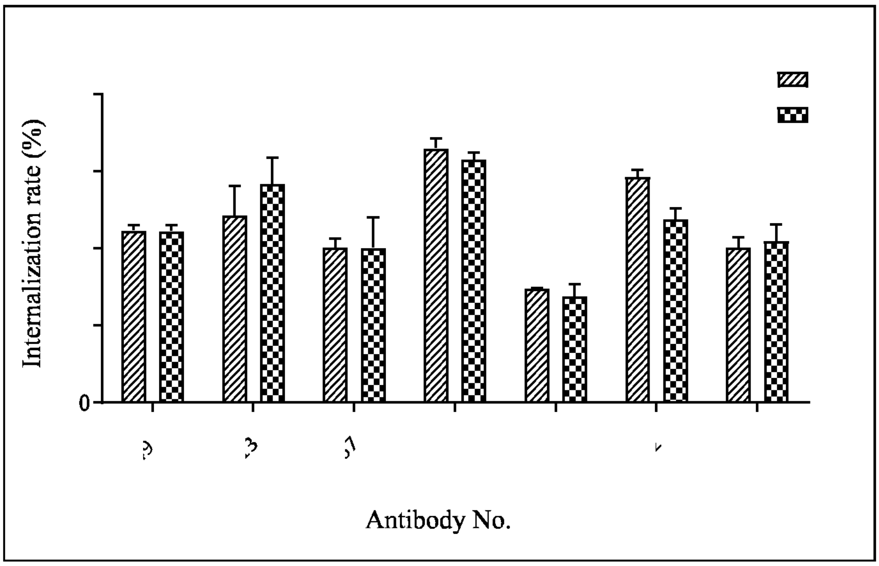


FIG. 5A

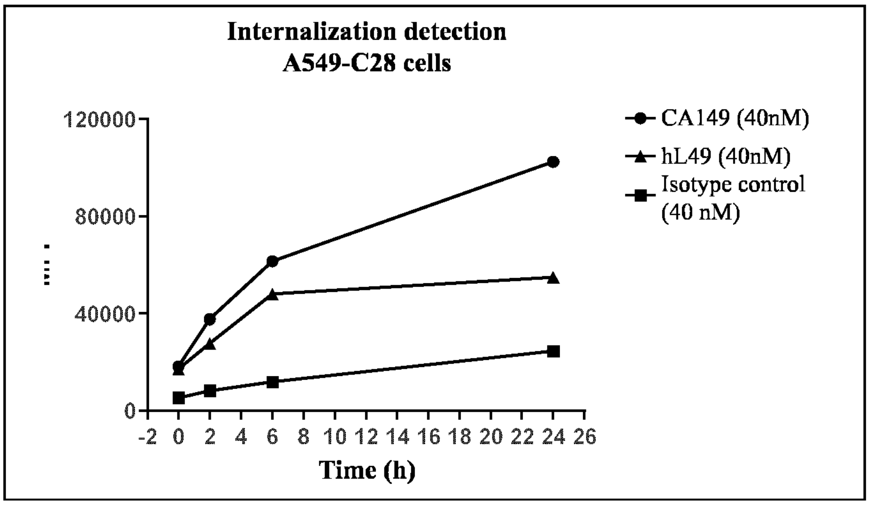


FIG. 5B

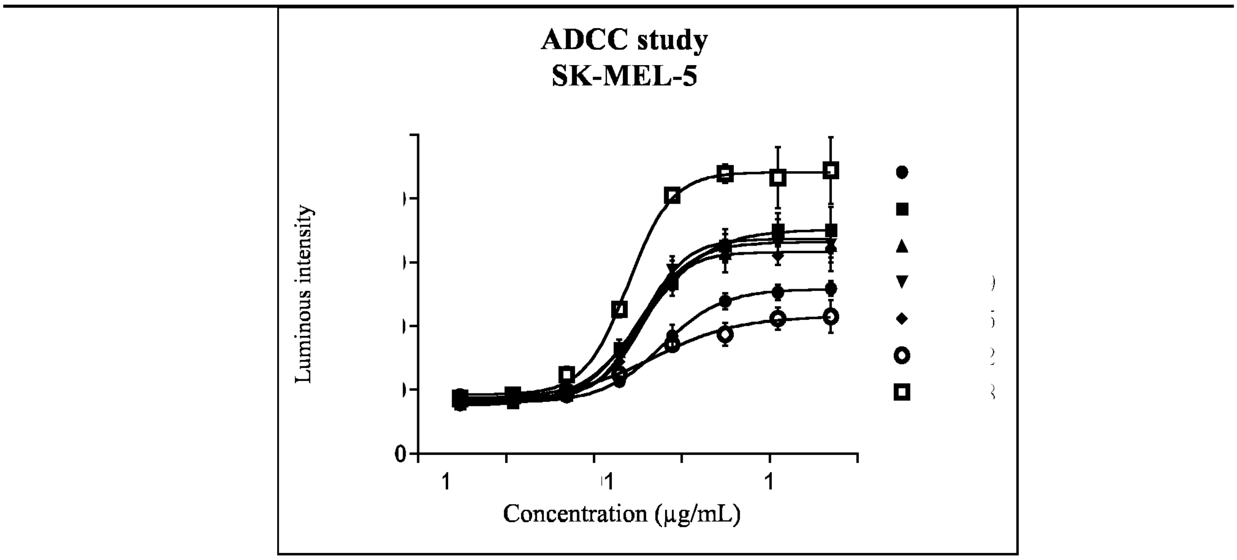
ADCC study
SK-MEL-5
Luminous intensity
0
1
1
1
Concentration (µg/mL)

F. G. 6

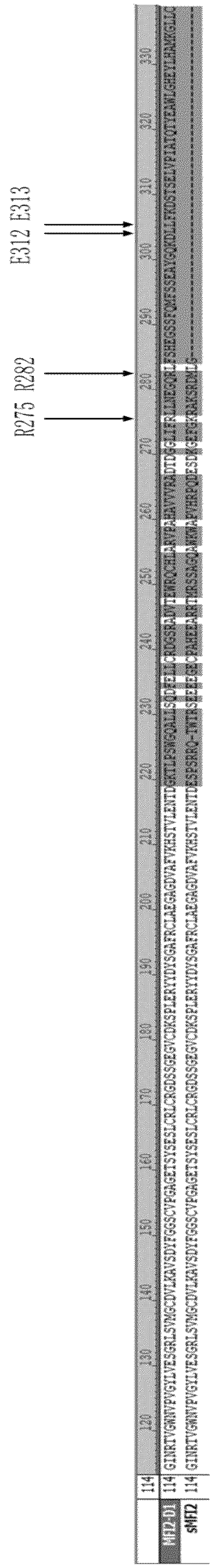


FIG. 7

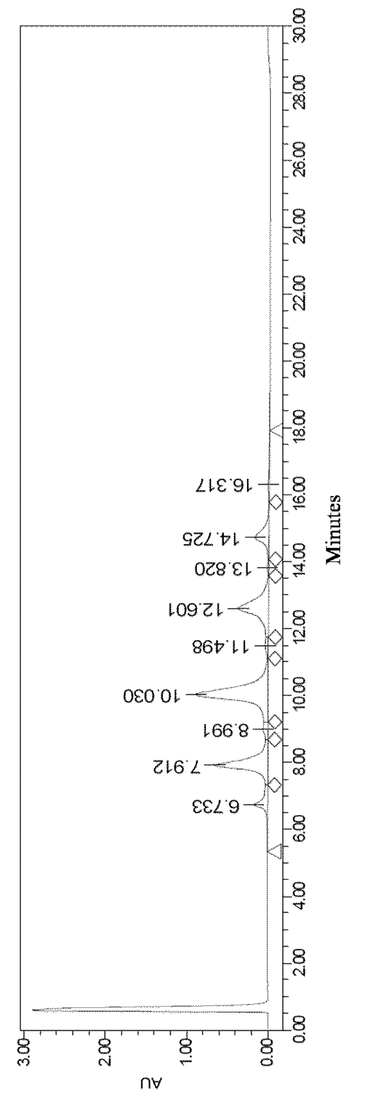
16.317
14.725
13.820
12.601
11.498
10.030
8.991
7.912
6.733
Minutes
AU
0.00
2.00
4.00
6.00
8.00
10.00
12.00
14.00
16.00
18.00
20.00
22.00
24.00
26.00
28.00
30.00
1.00
2.00
3.00

FIG. 8

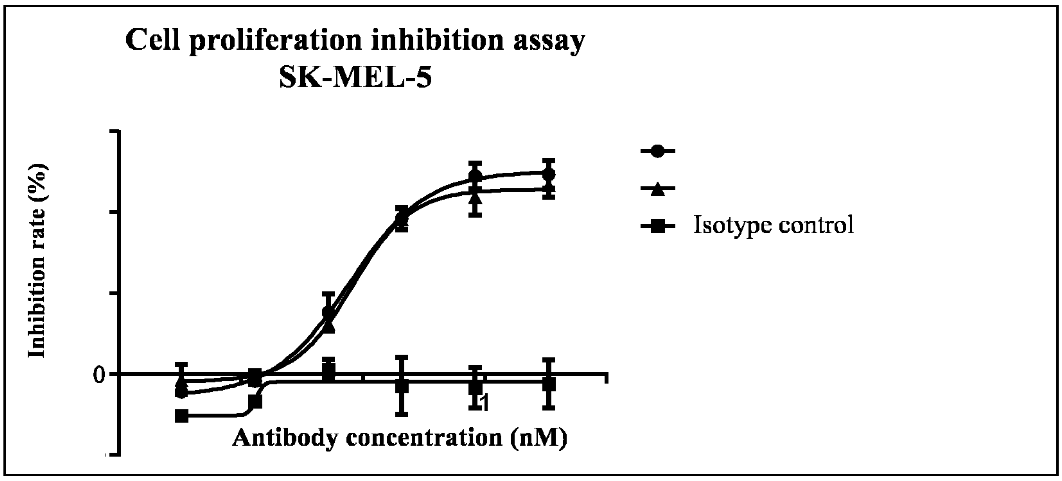
Cell proliferation inhibition assay
SK-MEL-5
Inhibition rate (%)
0
Antibody concentration (nM)
Isotype control

FIG. 9A

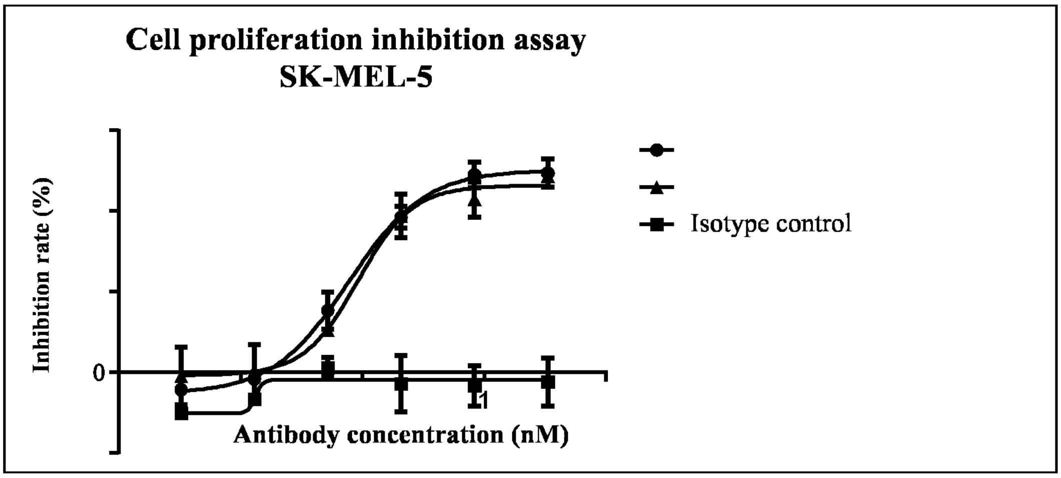


FIG. 9B

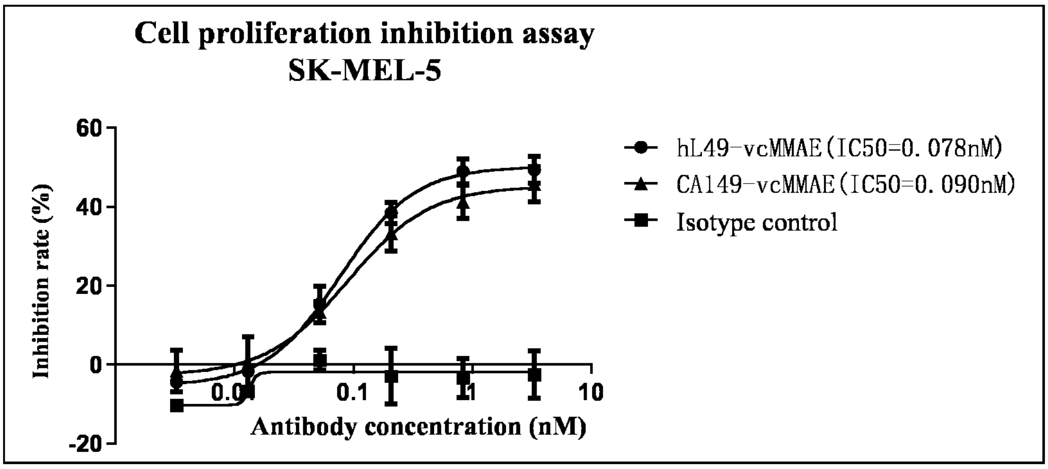


FIG. 9C

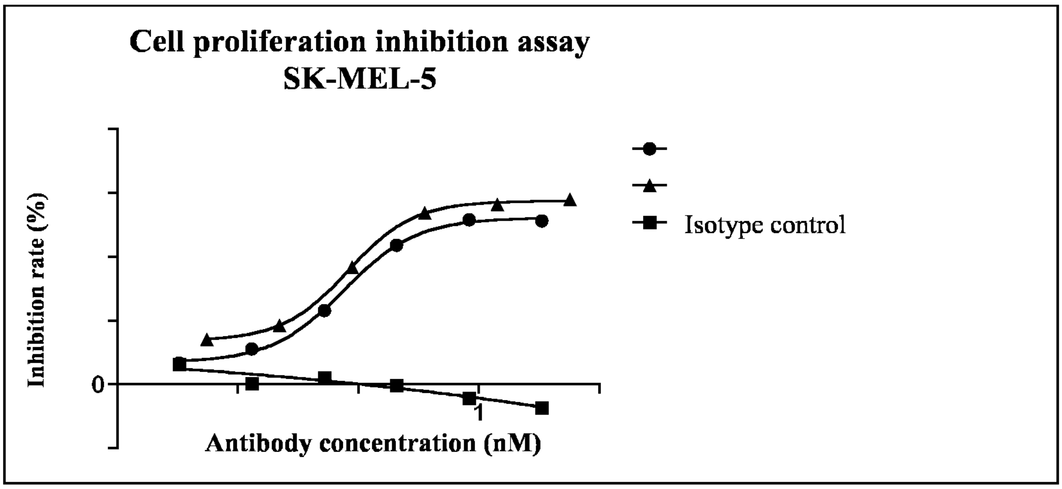


FIG. 9D

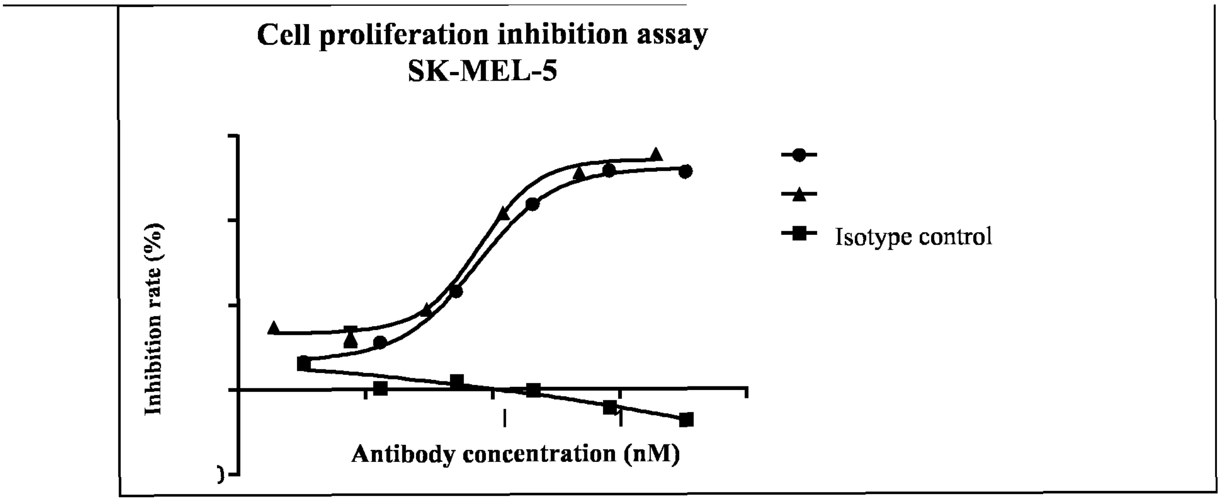


FIG. 9E

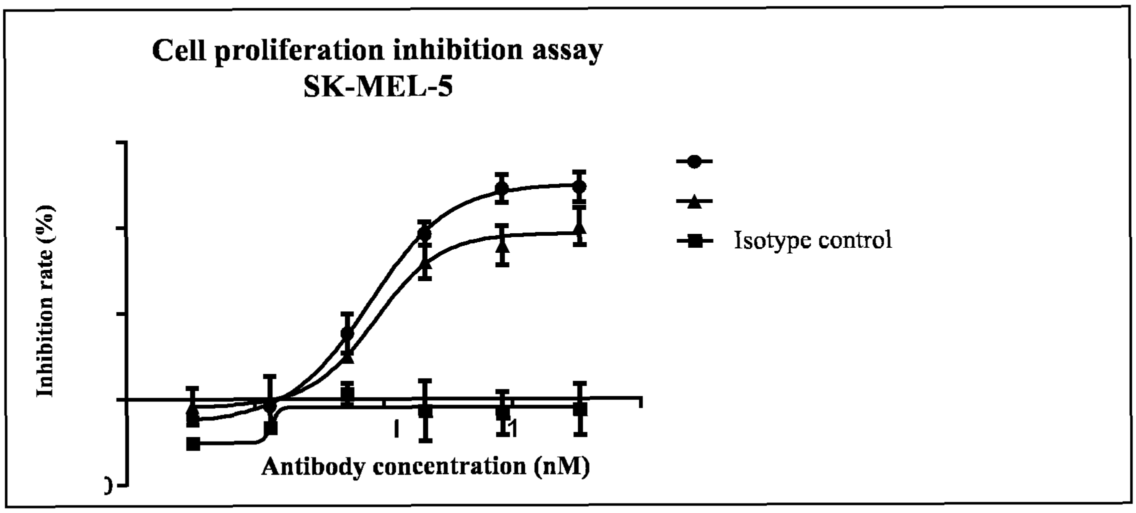


FIG. 9F

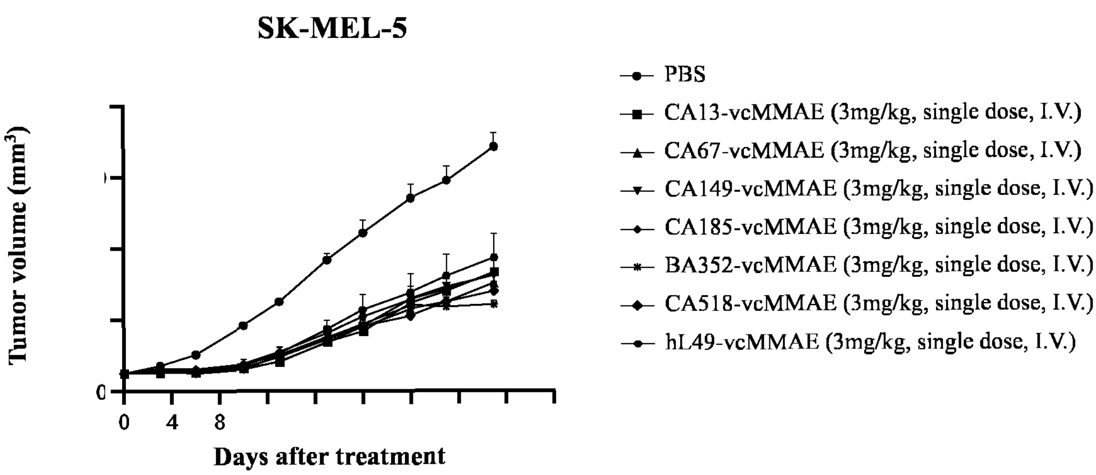


FIG. 10

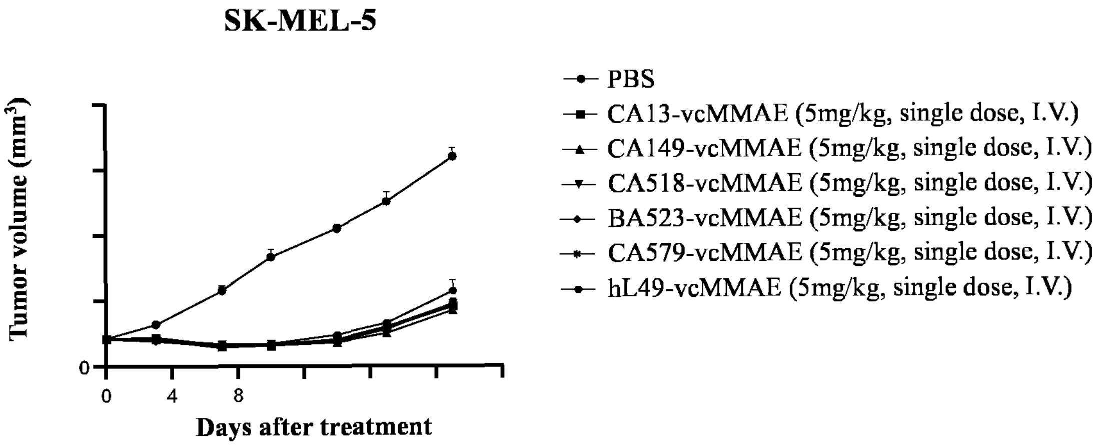


FIG. 11

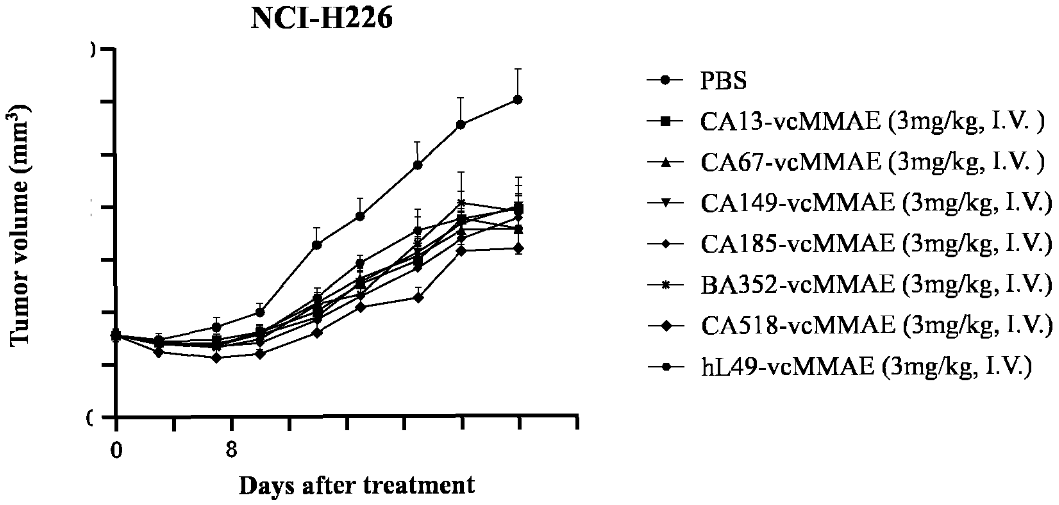


FIG. 12

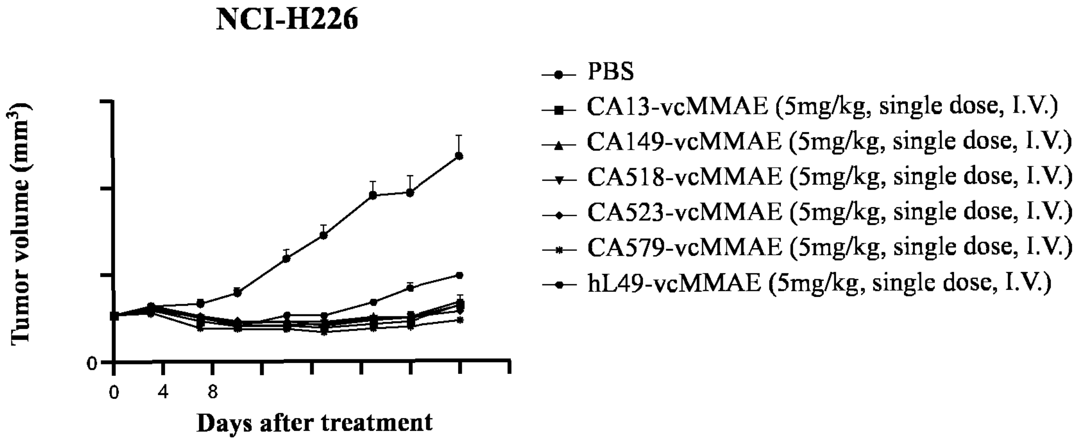


FIG. 13

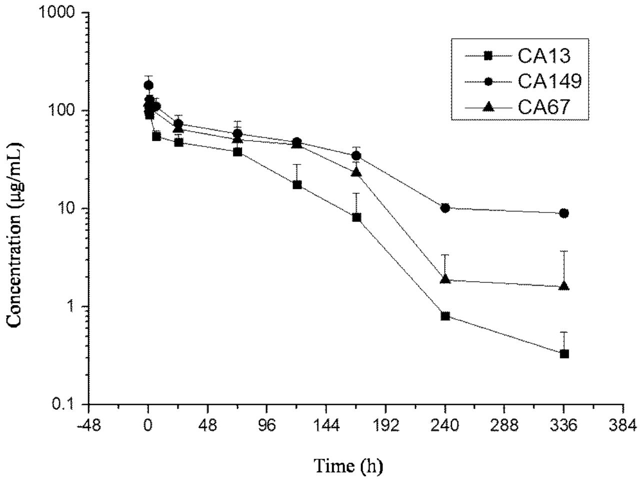


FIG. 14

**Cell proliferation inhibition assay**
**MC38-CD228 96h**

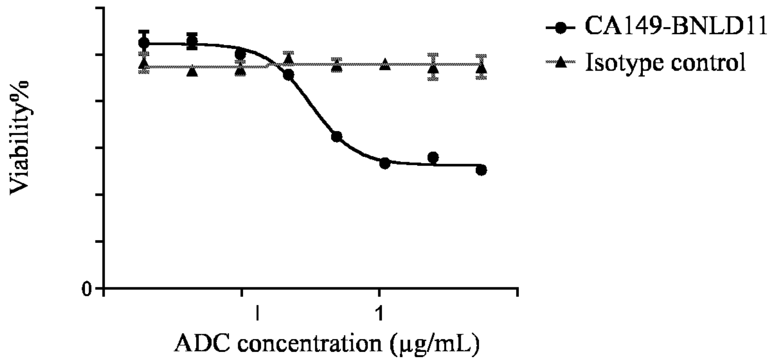


FIG. 15A

**Cell proliferation inhibition assay**
**A375-CD228 96h**

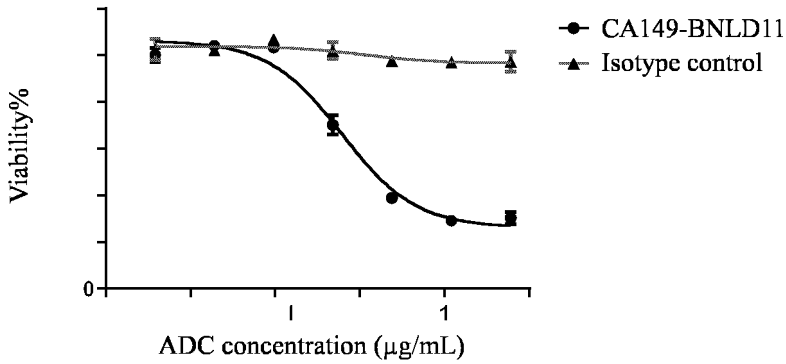


FIG. 15B

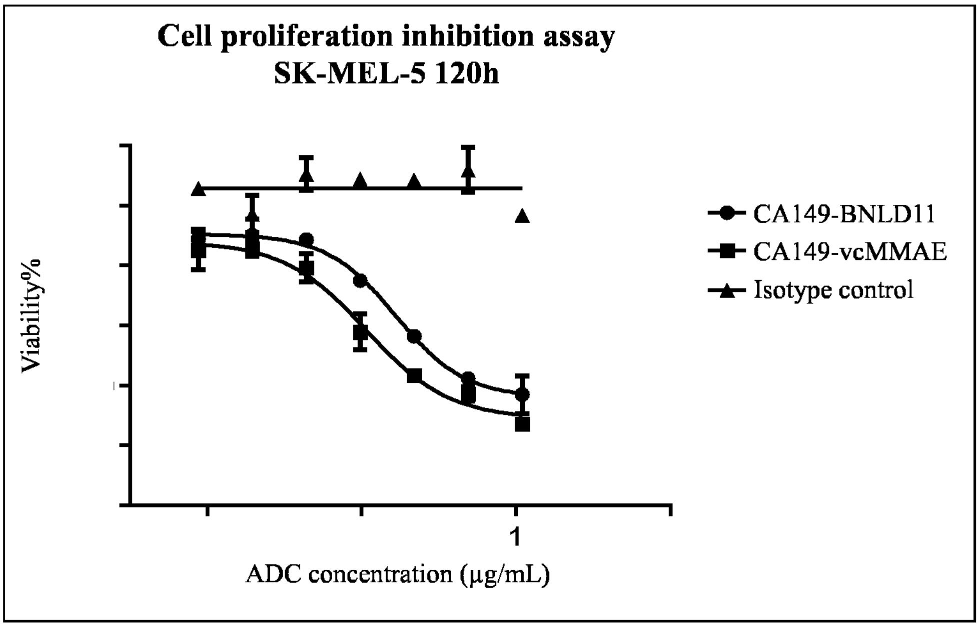


FIG. 15C

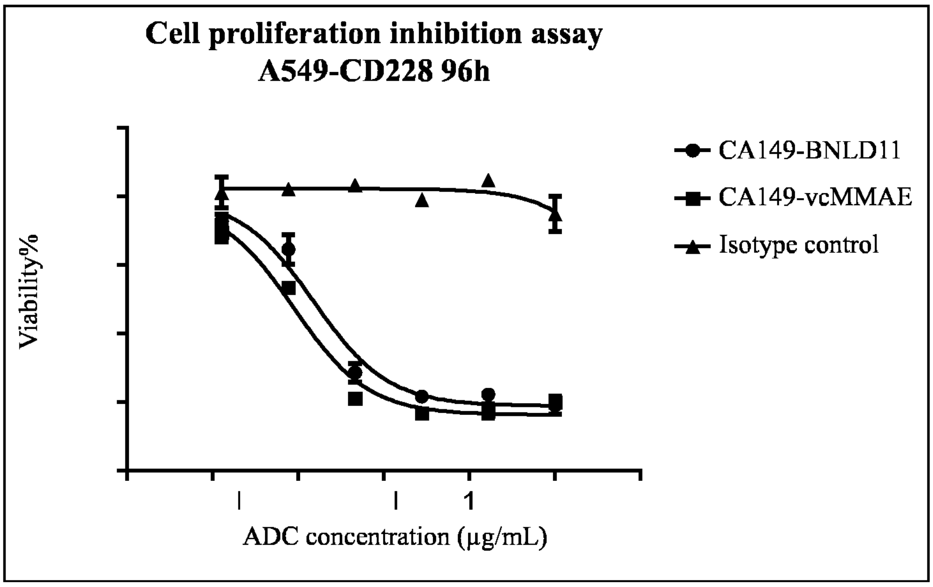


FIG. 15D

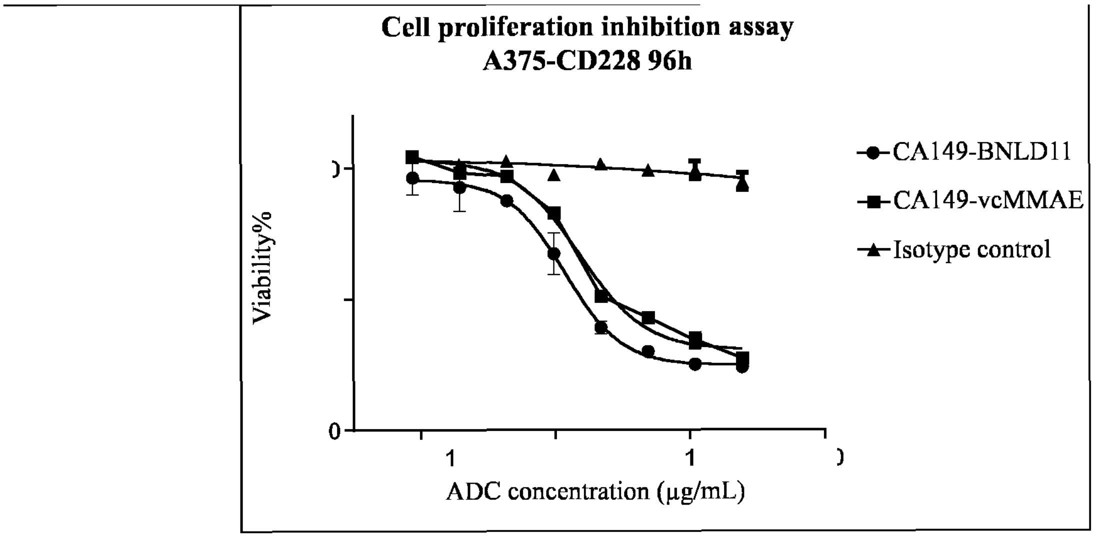


FIG. 15E

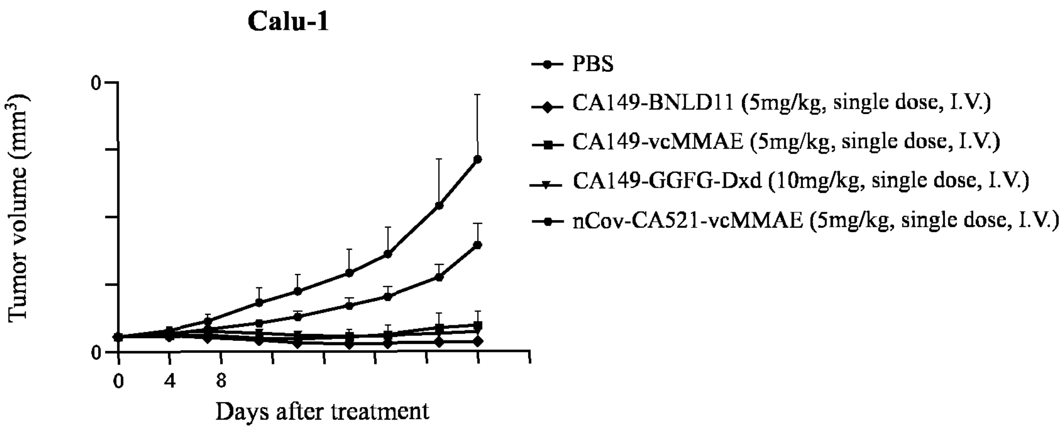


FIG. 16

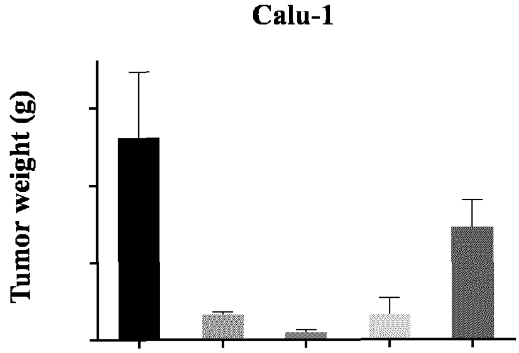


FIG. 17

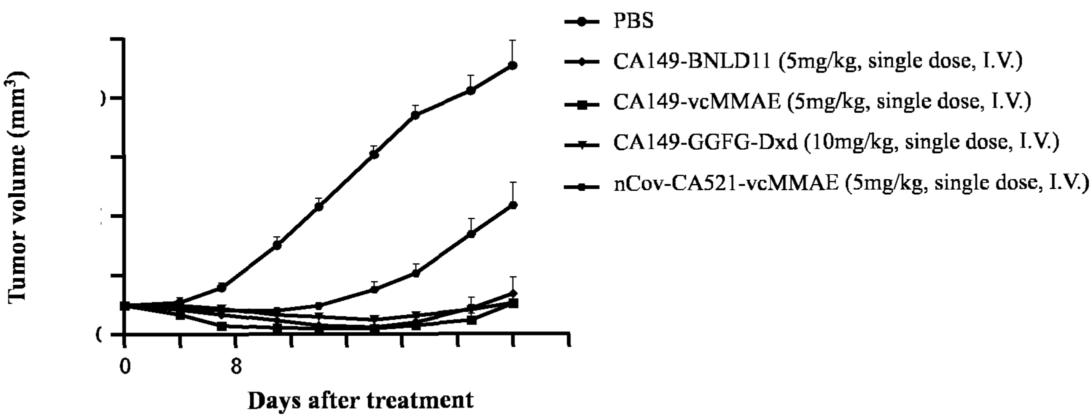


FIG. 18

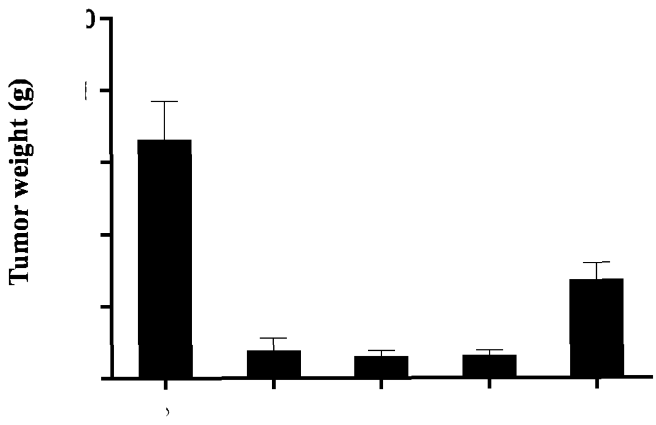


FIG. 19

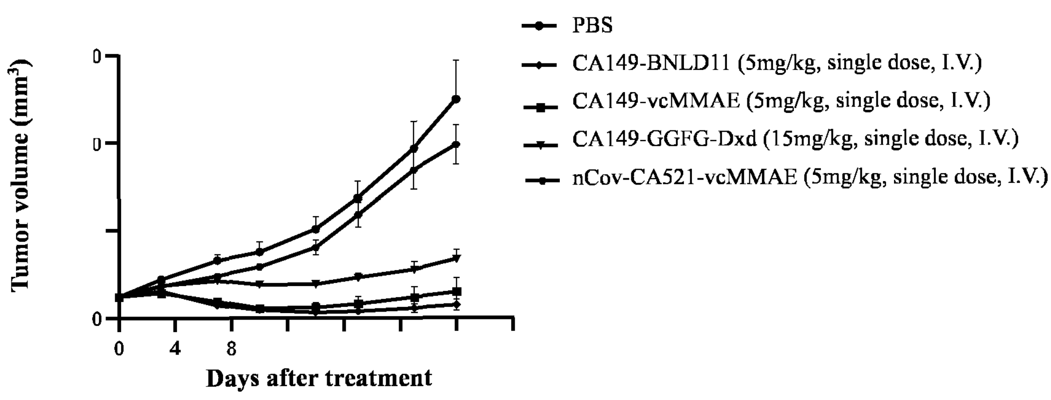


FIG. 20

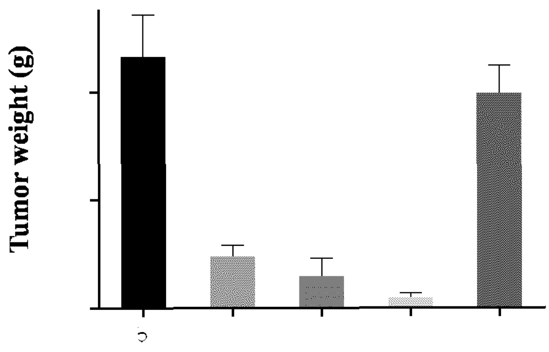


FIG. 21

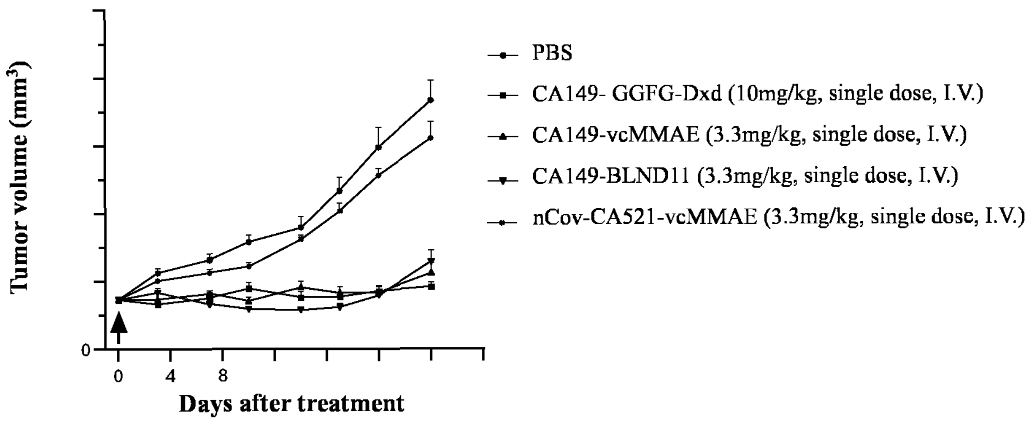


FIG. 22

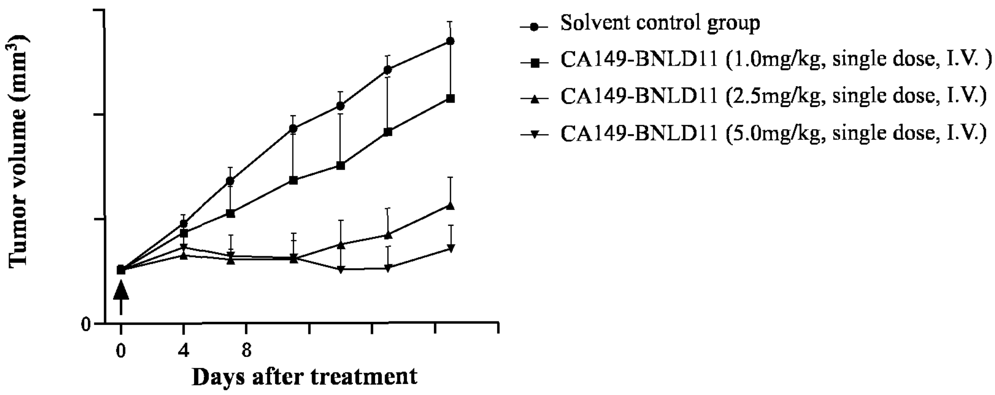


FIG. 23

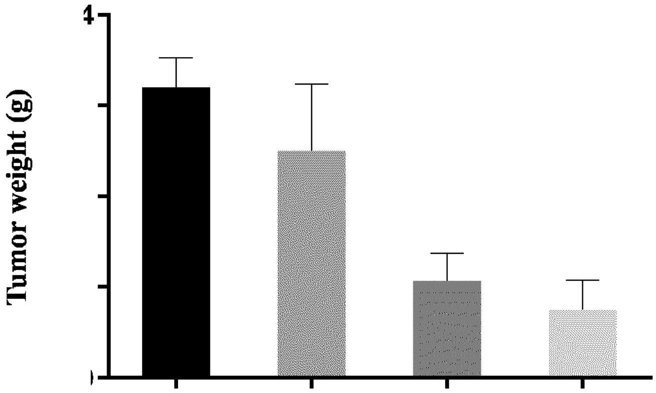


FIG. 24

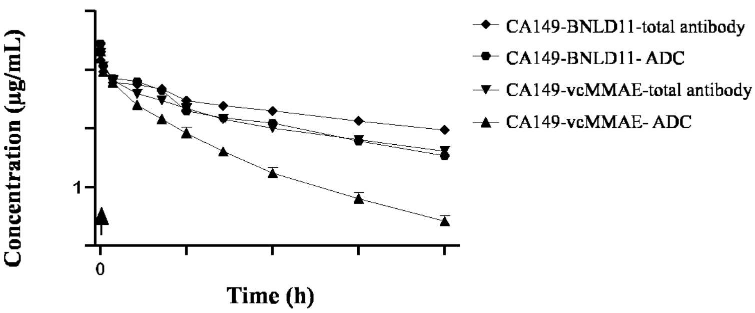


FIG. 25

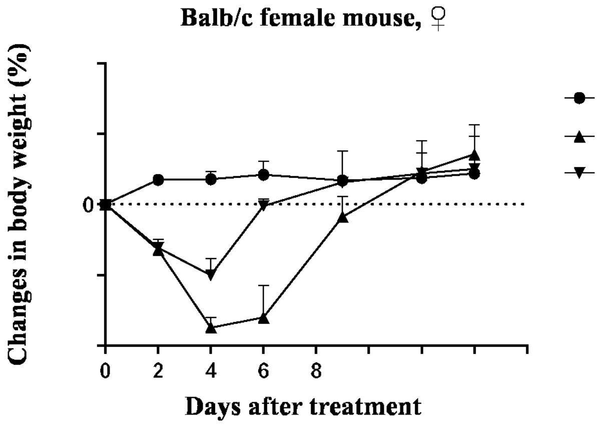


FIG. 26

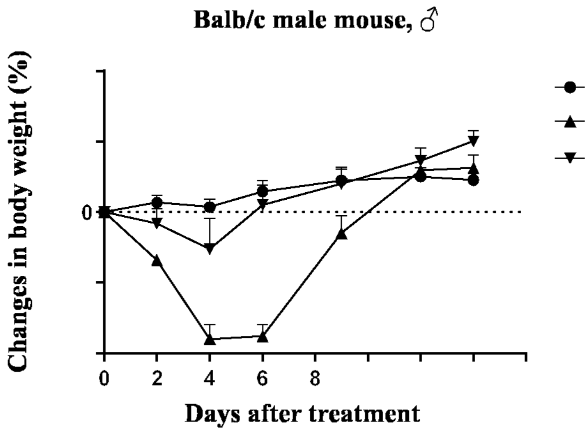


FIG. 27

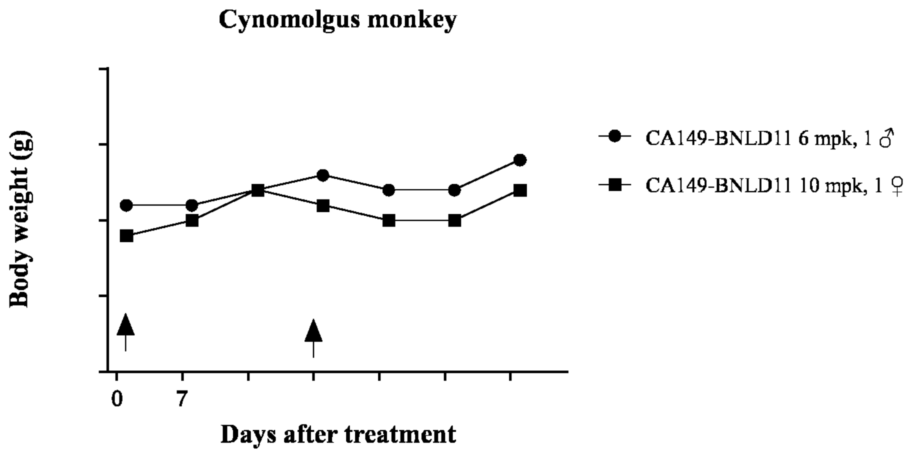


FIG. 28

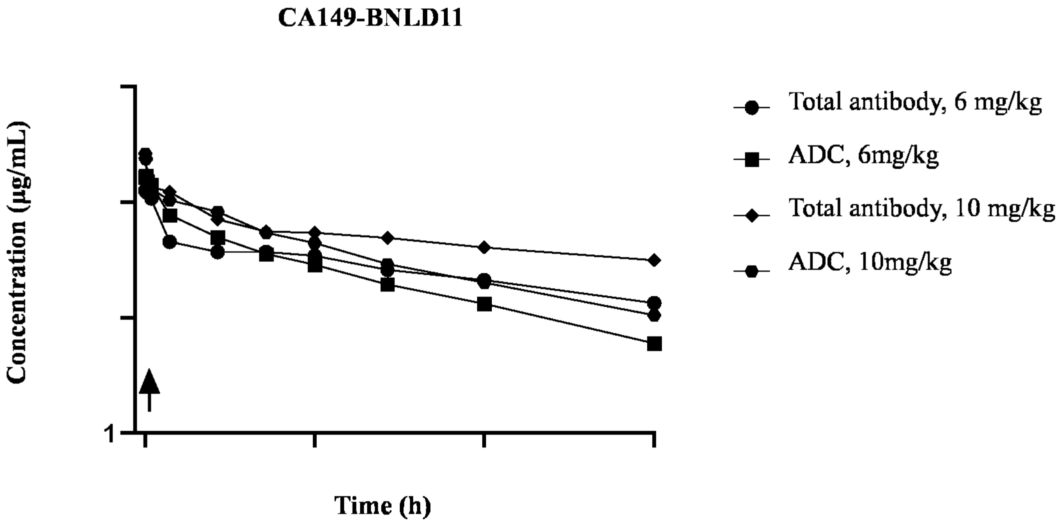


FIG. 29

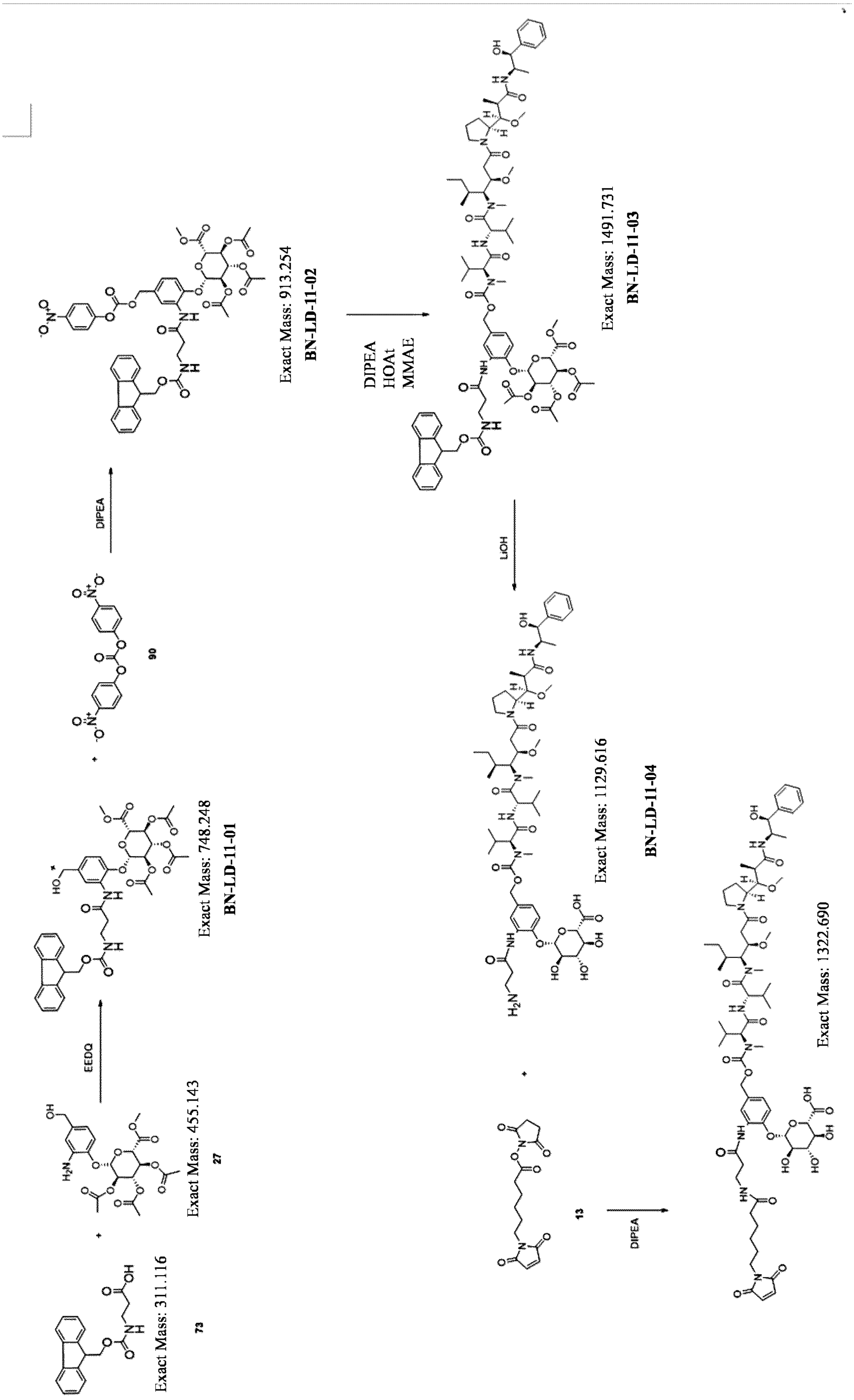
Exact Mass: 311.116
73
Exact Mass: 455.143
27
EEDQ
Exact Mass: 748.248
BN-LD-11-01
90
DIPEA
Exact Mass: 913.254
BN-LD-11-02
DIPEA
HOAt
MMAE
Exact Mass: 1491.731
BN-LD-11-03
LiOH
Exact Mass: 1129.616
BN-LD-11-04
13
DIPEA
Exact Mass: 1322.690

FIG. 30

## INTERNATIONAL SEARCH REPORT

International application No.
**PCT/CN2023/096155**

**A. CLASSIFICATION OF SUBJECT MATTER**

C07K16/28 (2006.01)i; C07K14/54 (2006.01)i; C07K16/18(2006.01)i; C07K16/30(2006.01)i; C12N 15/13(2006.01)i; A61K39/395(2006.01)i; A61K47/68(2017.01)i; G01N33/68 (2006.01)i; G01N33/574 (2006.01)i; G01N33/577 (2006.01)i; A61P35/00 (2006.01)i

According to International Patent Classification (IPC) or to both national classification and IPC

**B. FIELDS SEARCHED**

Minimum documentation searched (classification system followed by classification symbols)

IPC: C07K C12N A61K G01N A61P

Documentation searched other than minimum documentation to the extent that such documents are included in the fields searched

Electronic data base consulted during the international search (name of data base and, where practicable, search terms used)

CNABS; CNTXT; CJFD; WPABS; WPABSC; ENTXTC; ENTXT; DWPI; CNKI; 万方, WANFANG; 读秀, DUXIU; Web of Science; STN; EMBL; NCBI; 中国专利生物序列检索系统, China Patents Biological Sequence Search System: 山东博安生物技术, 抗体, 单抗, mAb, CD228, MTF, MELTF, p97, MFI2, MAP97, 黑色素转铁蛋白, 黑素瘤相关抗原, 抗体药物偶联物, 抗体药物缀合物, ADC, SHANDONG BOAN BIOTECHNOLOGY, antibody, mAb, melanotransferrin, melanoma-associated antigens, antibody-drug conjugates, SEQ ID NOs: 1-49

**C. DOCUMENTS CONSIDERED TO BE RELEVANT**

| Category* | Citation of document, with indication, where appropriate, of the relevant passages | Relevant to claim No. |
|---|---|---|
| A | CN 113710271 A (SEATTLE GENETICS, INC.) 26 November 2021 (2021-11-26) claims 1-30 and 88-89, and description, paragraphs [0215] and [0229] | 1-12 |
| A | WO 2022031652 A1 (SEAGEN INC.) 10 February 2022 (2022-02-10) claims 1-116 | 1-12 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 September 2023** | **15 September 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/096155**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/096155**

| Patent document cited in search report | Publication date (day/month/year) | Patent family member(s) | Publication date (day/month/year) |
|---|---|---|---|
| CN 113710271 A | 26 November 2021 | EP 3920968 A1 | 15 December 2021 |
| | | TW 202045536 A | 16 December 2020 |
| | | MX 2021009138 A | 10 September 2021 |
| | | AU 2020219732 A1 | 05 August 2021 |
| | | CA 3128097 A1 | 13 August 2020 |
| | | US 2020246479 A1 | 06 August 2020 |
| | | US 11617798 B2 | 04 April 2023 |
| | | JP 2022519273 A | 22 March 2022 |
| | | WO 2020163225 A1 | 13 August 2020 |
| | | KR 20210125511 A | 18 October 2021 |
| | | IL 284974 A | 30 September 2021 |
| WO 2022031652 A1 | 10 February 2022 | CA 3189225 A1 | 10 February 2022 |
| | | AR 124558 A1 | 12 April 2023 |
| | | KR 20230042518 A | 28 March 2023 |
| | | TW 202221034 A | 01 June 2022 |
| | | AU 2021320739 A1 | 23 February 2023 |
| | | EP 4192871 A1 | 14 June 2023 |
| | | IL 300176 A | 01 March 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20200246479 A1
- CN 202180003751
- DD 110101


### Non-patent literature cited in the description


- *J Neurochem.*, November 2002, vol. 83 (4), 924-33
- *J Cereb Blood Flow Metab.*, October 2019, vol. 39 (10), 2074-2088